# EUROPEAN PATENT SPECIFICATION

(11) **EP 3 728 567 B1**
(45) Date of publication and mention of the grant of the patent: **05.07.2023**
(21) Application number: 18893211.5
(22) Date of filing: 19.12.2018
(51) Int. Cl.: G01N 33/68, G16H 10/20, G16H 10/40, G16H 50/20, G16H 50/30, G16H 50/50, G16H 50/70

(54) **METHOD FOR ASSESSING A SYNUCLEINOPATHY**
VERFAHREN ZUR BEURTEILUNG EINER SYNUCLEINOPATHIE
MÉTHODE D'ÉVALUATION D'UNE SYNUCLÉINOPATHIE

(30) Priority: 19.12.2017 US 201762607345 P; 08.10.2018 US 201862742839 P; 19.11.2018 US 201862769255 P
(43) Date of publication of application: 28.10.2020
(73) Proprietor: Chase Therapeutics Corporation, Washington, DC 20006 (US)
(72) Inventor: CHASE, Thomas N., Washington, District of Columbia 20006 (US); CLARENCE-SMITH, Kathleen, Washington, District of Columbia 20006 (US)
(74) Representative: Patent Boutique LLP
(86) International application number: PCT/US2018/066612
(87) International publication number: WO 2019/126395

(56) References cited:
- WO-A1-2013/138512
- WO-A1-2015/200851
- WO-A1-2017/032871
- WO-A2-2015/068075
- US-A1- 2006 198 851
- US-A1- 2008 220 449
- US-A1- 2015 119 278
- US-A1- 2015 119 278
- ALEXANDER G. THOMPSON ET AL: "Extracellular vesicles in neurodegenerative disease - pathogenesis to biomarkers", NATURE REVIEWS. NEUROLOGY, vol. 12, no. 6, 13 May 2016 (2016-05-13), pages 346-357, XP055454065, US ISSN: 1759-4758, DOI: 10.1038/nrneurol.2016.68
- LÖÖV CAMILLA ET AL: "[alpha]-Synuclein in Extracellular Vesicles: Functional Implications and Diagnostic Opportunities", CELLULAR AND MOLECULAR NEUROBIOLOGY, SPRINGER NEW YORK, US, vol. 36, no. 3, 18 March 2016 (2016-03-18) , pages 437-448, XP035904163, ISSN: 0272-4340, DOI: 10.1007/S10571-015-0317-0 [retrieved on 2016-03-18]
- KIRA S. SHEINERMAN ET AL: "Circulating brain-enriched microRNAs as novel biomarkers for detection and differentiation of neurodegenerative diseases", ALZHEIMER'S RESEARCH & THERAPY, vol. 9, no. 1, 9 November 2017 (2017-11-09), XP055542774, DOI: 10.1186/s13195-017-0316-0
- MIN SHI ET AL: "Plasma exosomal [alpha]-synuclein is likely CNS-derived and increased in Parkinson's disease", ACTA NEUROPATHOLOGICA, vol. 128, no. 5, 6 July 2014 (2014-07-06), pages 639-650, XP055225837, BERLIN, DE ISSN: 0001-6322, DOI: 10.1007/s00401-014-1314-y
- JINGTI DENG ET AL: "Neurons Export Extracellular Vesicles Enriched in Cysteine String Protein and Misfolded Protein Cargo", SCIENTIFIC REPORTS, vol. 7, no. 1, 19 April 2017 (2017-04-19), page 956, XP055755654, DOI: 10.1038/s41598-017-01115-6
- REN et al.: "Characteristics of tau oligomers", Frontiers in Neurology, vol. 4, 19 July 2013 (2013-07-19), XP055251642, DOI: doi:10.3389/fneur.2013.00102
- Anonymous: "Machine learning", Wikipedia, 7 November 2017 (2017-11-07), XP055620396, Retrieved from the Internet: URL:https://en.wikipedia.org/w/index.php?t itle=Machine-learning&oldid=809126985 [retrieved on 2019-02-25]
- "Alpha-synuclein oligomers: a new hope", Acta Neuropathol, vol. 134, 12 August 2017 (2017-08-12), page 819.838, XP036351197,
- Anonymous: "Clinical Global Impression", Wikipedia, 7 February 2017 (2017-02-07), pages 1-3, XP055620400, Retrieved from the Internet: URL:https://en.wikipedia.org/w/index.php?t itle=Clinical_Global_lmpression&oldid=7642 63911 [retrieved on 2019-02-25]
- SENGUPTA et al.: "The Role of Amyloid-[beta] Oligomers in Toxicity, Propagation, and Immunotherapy", EBioMedicine, vol. 6, no. 2016 5 April 2016 (2016-04-05), pages 42-49, XP055620404, Retrieved from the Internet: URL:http://dx.doi.org/10.1016/j.ebiom.2016 .03.035

## Description

### BACKGROUND

Neurodegenerative diseases are characterized by degenerative changes in the brain including loss of function and death of neurons. Neurodegenerative diseases include, without limitation, Parkinson's disease, Alzheimer's disease, Huntington's disease, amyotrophic lateral sclerosis and Lewy Body dementia.

Many neurodegenerative diseases are characterized by the aberrant accumulation of oligomeric forms of proteins. It is believed that these oligomeric forms contribute to neuronal degeneration and death. US 2015/0119278 A1 describes biomarkers and diagnostic and prognostic methods for Alzheimer's disease and other neurodegenerative disorders. WO 2015/200851 A1 describes a method for enriching CNS derived exosomes from a biological fluid and that biomarkers from the CNS derived exosomes can be measured for detecting a neurological disease, differentiating among different neurological diseases, monitoring disease progression or objectively assessing existing and future medical treatments. WO 2015/068075 A2 describes an *in vitro* method for quantifying Tau protein in an individual comprising measuring the amount of Tau protein in an Ectosome-Enriched Fraction from said individual. WO 2017/032871 A1 describes methods of differential diagnosis of dementia with Lewy bodies and/or Parkinson's disease comprising a step of isolating exosomes from a sample of cerebrospinal fluid (CSF) and determining the number of exosomes and/or amount of α-Synuclein in a defined volume of CSF sample. "Extracellular vesicles in neurodegenerative disease - pathogenesis to biomarkers" Thompson et al, Nature Reviews Neurology, volume 12, June 2016 pg. 346-357 describes the biology and function of extracellular vesicles, discusses the evidence for their involvement in the pathogenesis of neurodegenerative diseases, and considers their potential as biomarkers of disease. "α-Synuclein in Extracellular Vesicles: Functional Implications and Diagnostic Opportunities" Lööv et al, Cell Mol. Neurobiol, 36, 2016, pg. 437-448, describes that α-synuclein may be transferred between cells via extracellular vesicles, the presence of extracellular vesicle associated α-synuclein may act as a diagnostic biomarker for Parkinson's disease and other α-synucleinopathies, and methods for detecting α-synuclein in extracellular vesicles. WO 2013/138512 A1 describes methods for diagnosis, prognosis and monitoring of Alzheimer's disease involving measuring the amounts of combined monomeric and oligomeric Aβ protein and amounts of monomeric Aβ protein in a sample obtained from a subject and determining a ratio so that the ratio can be used in diagnosing, prognosing and/or monitoring Alzheimer's disease. US 2008/0220449 A1 describes a method for diagnosis, stratification and monitoring progression of Alzheimer's disease which is based on the quantification of tau oligomers in extracellular CSF using a modified ELISA assay. "Circulating brain-enriched microRNAs as novel biomarkers for detection and differentiation of neurodegenerative diseases" Sheinerman et al, Alzheimer's Research & Therapy, (2017), 9:89, describes the possibility of developing microRNA-based diagnostics for detection and differentiation of neurodegenerative diseases.

### SUMMARY

The invention provides, as specified by the claims, a method of inferring a risk of developing, a diagnosis of, a stage of, a prognosis of, a progression of, or a degree of response to a putative neuroprotective agent for a synucleinopathy. Disclosed herein are, among other things, biomarker profiles for neurodegenerative conditions, such as synucleopathic conditions, amyloidopathic conditions, tauopathies and Huntington's disease, and the neurodegeneration associated therewith. The biomarker profile comprises measures of one or a plurality of different species (also referred to as "forms") of alpha-synuclein. The alpha-synuclein profile can comprise quantitative measures of each of one or a plurality of neurodegenerative protein forms are selected from: (I) at least one oligomeric form; (II) a plurality of oligomeric forms; (III) at least one oligomeric form and at least one monomeric form; (IV) a plurality of oligomeric forms and at least one monomeric form; (V) at least one oligomeric form and a plurality of monomeric forms; and (VI) a plurality of oligomeric forms and a plurality of monomeric forms.

, The protein species are measured from CNS-derived extracellular vesicles, hereinafter termed exosomes, isolated, from blood. The species examined can derive from an internal compartment of the exosome, e.g., from exosomes from which surface proteins have been removed. The biomarker profiles, measured in this way, represent a relatively simple and non-invasive means for measurement.

As such, methods of this disclosure for measuring a biomarker profile for neurodegeneration are useful in drug development for testing neuroprotective efficacy of a drug candidate, sometimes referred to herein as a putative neuroprotective agent. For example, the methods described herein can be used to further understand the downstream effects and molecular basis of oligomerization in neurodegenerative conditions, such as synucleinopathies, and to accelerate the development of effective therapeutic strategies. Such methods also are useful for identifying subjects for enrollment in clinical trials and for determining a diagnosis, prognosis, progression or risk of developing a synucleopathic condition. Further described herein are novel methods of treating a subject determined, by the methods of this disclosure, to have or to be at risk of developing neurodegeneration associated with synucleopathic conditions, in particular, a neuroprotective treatment (not claimed).

### BRIEF DESCRIPTION OF THE DRAWINGS

The novel features of the disclosure are set forth with particularity in the appended claims. A better understanding of the features and advantages of the present disclosure will be obtained by reference to the following detailed description that sets forth illustrative embodiments, in which the principles of the disclosure are utilized, and the accompanying drawings of which:

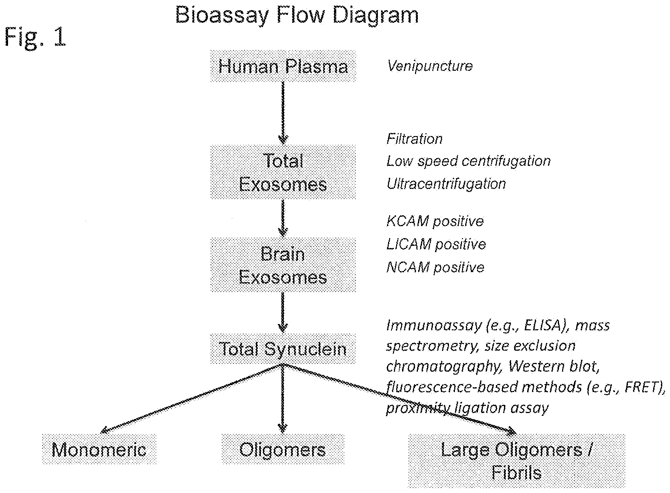

**FIG. 1** shows a flow diagram of an exemplary method detecting monomeric and oligomeric forms of alpha-synuclein.

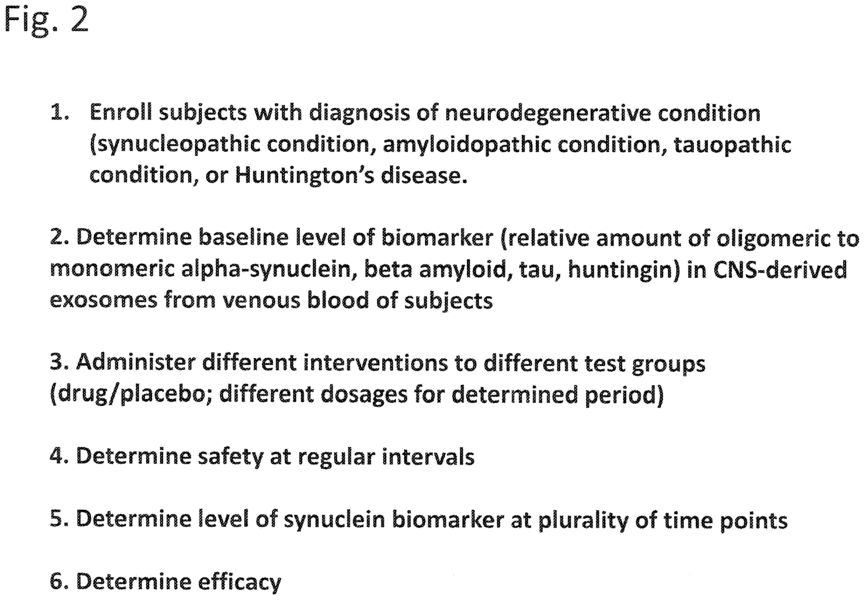

**FIG. 2** shows a flow diagram of an exemplary protocol to validate drug efficacy.

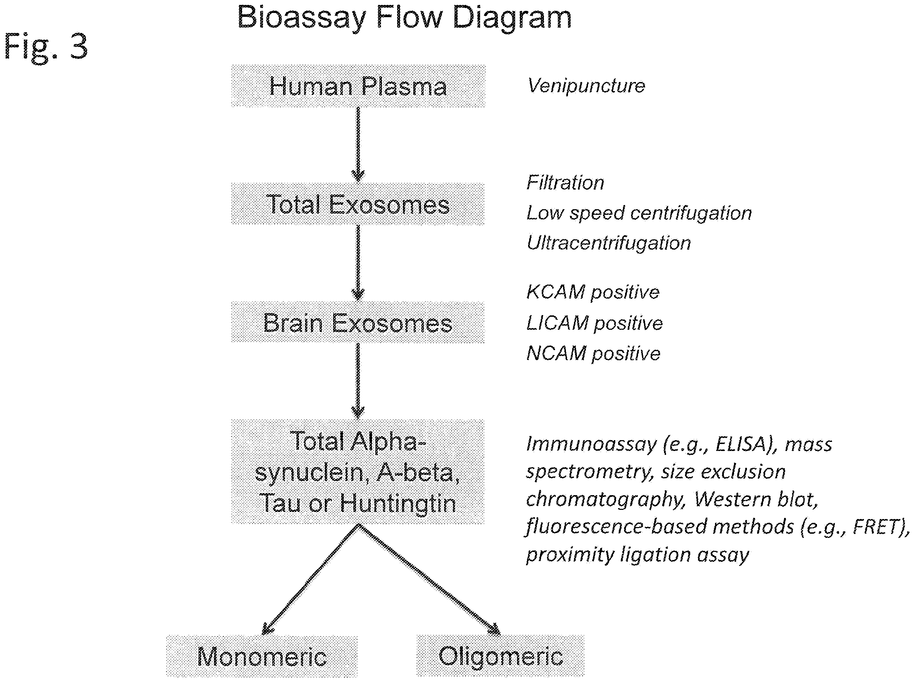

**FIG. 3** shows a flow diagram of an exemplary method detecting monomeric and oligomeric forms of proteins involved in neurodegenerative conditions.

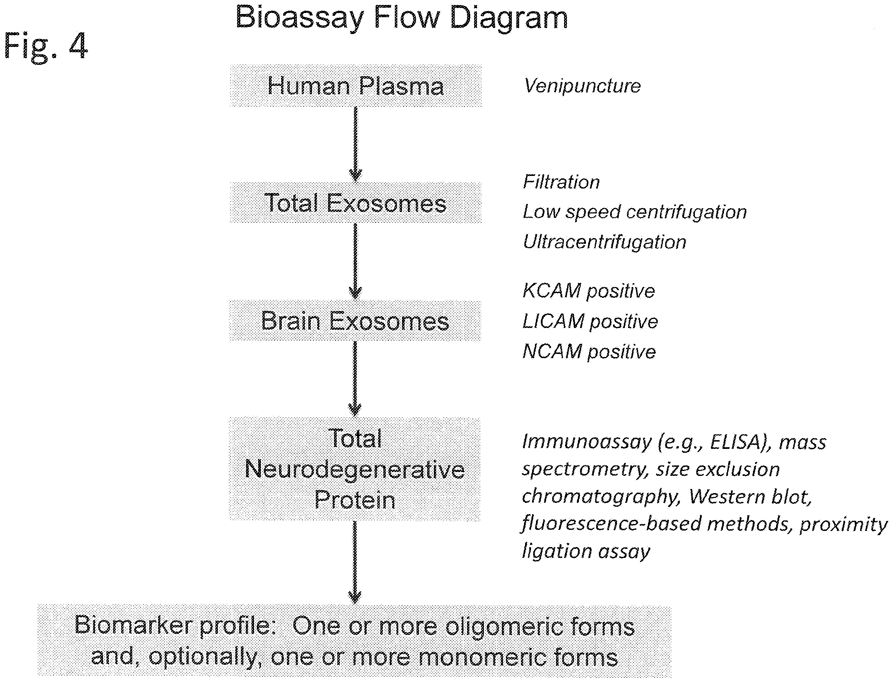

**FIG. 4** shows a flow diagram of an exemplary method detecting monomeric and oligomeric forms of a neurodegenerative protein.

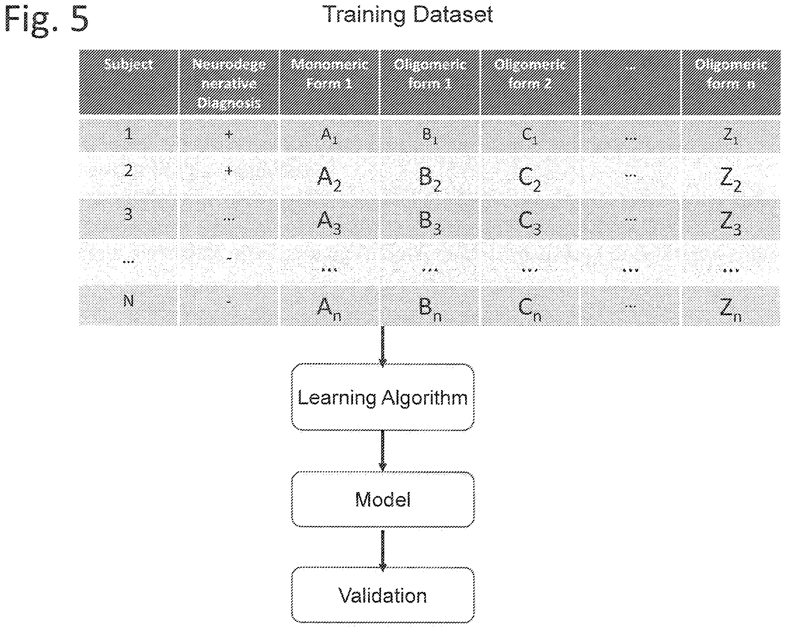

**FIG. 5** shows an exemplary flow diagram of creating and validating a diagnostic model for diagnosing a neurodegenerative condition.

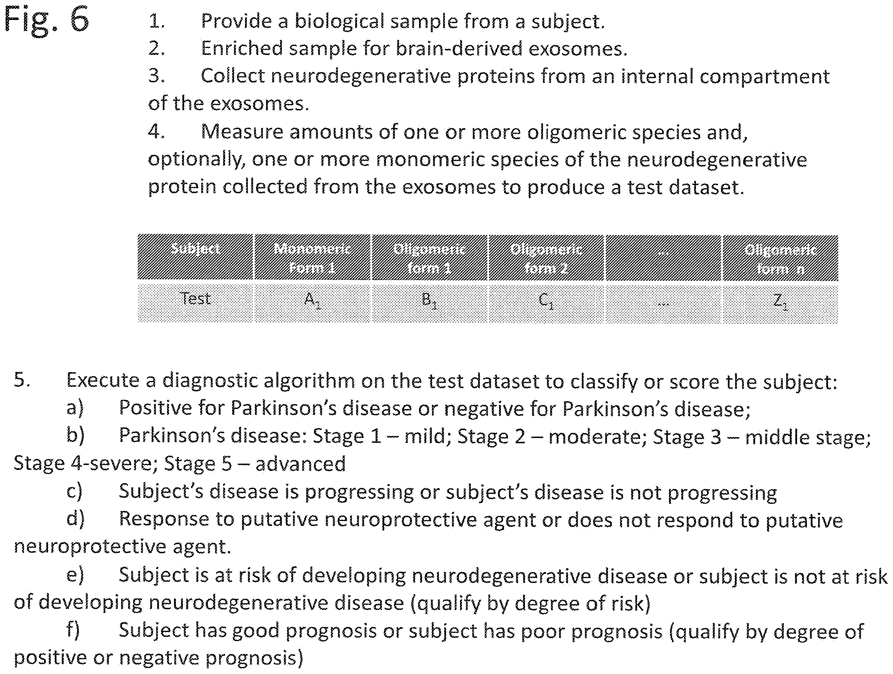

**FIG. 6** shows an exemplary flow diagram for classifying a subject according to any of several states by executing a diagnostic algorithm, or model, on a biomarker profile.
**FIG. 7** shows exemplary biomarker profiles including monomeric and five oligomeric species of alpha-synuclein in five different states. Such profiles can be used to correlate with various states. Profiles can be used by models executed by a human operator or by a computer.

### DETAILED DESCRIPTION

### I. Neurodegenerative Conditions and Associated Proteins

Methods disclosed herein are useful for diagnosis of and drug development for a variety of neurodegenerative conditions. These include, without limitation, synucleinopathies (e.g., Parkinson's disease, Lewy body dementia, multiple system atrophy), amyloidopathies (e.g., Alzheimer's disease), tauopathies (e.g., Alzheimer's disease, Progressive supranuclear palsy, Corticobasal degeneration), and Huntington's disease. These diseases share in common the accumulation of toxic oligomeric polypeptide species, and in some cases abnormally phosphorylated oligomeric or monomeric forms, and the ability to detect such forms in CNS-derived exosomes.

As used herein, the term "neurodegenerative protein" refers to a protein which, in an oligomerized form, is associated with neurodegeneration. Neurodegenerative proteins include, without limitation, alpha-synuclein, tau, amyloid beta and huntingtin.

It is believed that certain oligomerized forms or abnormally phosphorylated forms of brain polypeptides underlie a variety of neurodegenerative conditions. This includes, for example, the roles of alpha-synuclein in synucleinopathic conditions, amyloid beta in amyloidopathic conditions, tau in tauopathic conditions and huntingtin in Huntington's disease. In particular, current evidence suggests that α-synuclein oligomers can act as a toxic species in PD and other synucleinopathies. In certain embodiments, the oligomeric species detected is an abnormally phosphorylated species.

Profiles including amounts of each of one or a plurality of neurodegenerative protein forms (e.g., forms of alpha-synuclein, amyloid beta, tau, or huntingtin) selected from: (I) at least one oligomeric form; (II) a plurality of oligomeric forms; (III) at least one oligomeric form and at least one monomeric form; (IV) a plurality of oligomeric forms and at least one monomeric form; (V) at least one oligomeric form and a plurality of monomeric forms; and (VI) a plurality of oligomeric forms are used in models to infer, among other things, neurodegenerative conditions or progression toward neurodegenerative conditions, typically with one or more oligomeric forms included in a model indicating the presence and activity of the disease or progression towards the disease. This includes increasing relative amounts of oligomeric alpha-synuclein forms indicating the presence and activity of a synucleinopathy, or progression towards a synucleinopathy; increasing relative amounts of oligomeric amyloid beta indicating the presence and activity of an amyloidopathy, or progression towards an amyloidopathy, increasing relative amounts of oligomeric or abnormally phosphorylated tau indicating the presence and activity of a tauopathy, or progression towards a tauopathy, and increasing relative amounts of oligomeric huntingin indicating the presence and activity of Huntington's disease, or progression towards Huntington's disease. Accordingly, an abnormal profile of such oligomers indicates a process of neurodegeneration.

As used herein, the term "biomarker profile" refers to data indicating quantitative measures of each of one or a plurality of neurodegenerative protein forms including one or more oligomeric forms and, optionally, one or more monomeric forms. This includes amounts of species of oligomeric and, optionally, monomeric alpha-synuclein; oligomeric and, optionally, monomeric amyloid beta, oligomeric and, optionally hyperphosphorylated and, optionally, monomeric tau; and oligomeric and, optionally, monomeric huntingtin. For example, a biomarker profile can include (I) at least one oligomeric form; (II) a plurality of oligomeric forms; (III) at least one oligomeric form and at least one monomeric form; (IV) a plurality of oligomeric forms and at least one monomeric form; (V) at least one oligomeric form and a plurality of monomeric forms; and (VI) a plurality of oligomeric forms and a plurality of monomeric forms.

Protein forms can refer to individual protein species or collections of species. For example, a 6-mer of alpha-synuclein is a form of alpha backspace-synuclein. Also, the collection of 6-mers to 18-mers of alpha-synuclein, collectively, can be a form of alpha-synuclein.

A biomarker profile can include a plurality of forms of a protein. In one embodiment, a biomarker profile can include quantitative measures of each of a plurality of oligomeric forms and monomeric form of the neurodegenerative protein. So, for example, the biomarker profile could include quantitative measures of each of a 6-mer, 7-mer, 8-mer, 9-mer, 10-mer, 11-mer, 12-mer, 13-mer, 14-mer, 15-mer, and 16-mer, 9-mer.

Quantitative measures can be absolute measures, normalized measures (e.g., against a reference measurement) and relative measures. For example, in one embodiment, a biomarker profile comprises a relative amount of an oligomeric form of a neurodegenerative protein to a monomeric form of the neurodegenerative protein.

The term "biomarker profile" may also be used to refer to a particular pattern in the profile which a model infers to be associated with a diagnosis, stage, progression, rate, prognosis, drug responsiveness and risk of developing a neurodegenerative condition. Accordingly, "synuclein biomarker profile" refers to a profile comprising oligomeric and, optionally, monomeric alpha-synuclein, the term "amyloid biomarker profile" refers to a profile comprising oligomeric and, optionally, monomeric beta-amyloid, the term "tau biomarker profile" refers to a profile comprising oligomeric and, optionally, monomeric tau, the term "huntintin biomarker profile" refers to a profile comprising oligomeric and, optionally, monomeric huntintin.

As used herein, the term "monomeric protein/polypeptide" refers to a single, non-aggregated protein or polypeptide molecule, including any species thereof, such as phosphorylated species. As used herein, the term "oligomeric protein/polypeptide" refers to individual oligomeric species or an aggregate comprising a plurality of oligomeric species, including phosphorylated species. It is understood that measurement of an oligomeric form of a protein, as used herein, can refer to measurement of all oligomeric forms (total oligomeric form) or specified oligomeric forms. Specified oligomeric forms can include, for example, forms within a particular size range or physical condition such as for example soluble fibrils.

Abnormal profiles, (e.g., increased relative amounts of oligomeric to monomeric forms or increases or decreases of certain oligomeric forms relative to other oligomeric forms) indicate pathologic activity, and thus time to future clinical onset and subsequent rates of clinical progression. Moreover, return toward normal in biomarker profiles (e.g., reductions in relative amounts of oligomeric forms to monomeric forms) reflects the efficacy of a candidate neuroprotective intervention. Accordingly, the biomarker profiles described herein are useful for determining efficacy of drug candidates for their neuroprotective effect.

Accordingly, biomarker profiles function not only as a diagnostic of an existing pathological state but also as a sentinel of pathology before clinical onset, e.g., when a subject is pre-symptomatic or preclinical, e.g., has signs or symptoms that are insufficient for a diagnosis of disease. This is relevant since the relative success of neuroprotective treatments often appear related to their earliest possible administration. Further, it is believed that these biomarker profiles indicate the stage or degree of a neurodegenerative condition. Accordingly, determining biomarker profiles (e.g., relative amounts of oligomeric and monomeric forms of the selected protein) is useful for determining effectiveness of a treatment, for example, in clinical trials and, for therapeutic interventions believed to be effective for treating neurodegeneration including, e.g., synucleinopathy, amyloidopathy, tauopathy or Huntington's disease in the individual.

In each of these conditions, it is believed that oligomerized/ aggregated forms of polypeptides described herein are toxic to neurons in that the biomarker profiles comprising oligomeric forms and, optionally, monomeric forms of these polypeptides function in models to infer pathologic activity. In particular, increased relative amounts of oligomeric forms as compared with monomeric forms indicate pathology. Measures of these biomarkers can be used to track subject responses to therapies that are either in existence or in development as well as to predict development of disease or the state or progress of existing disease.

### A. Synucleinopathies

### 1. Conditions

As used herein, the terms "synucleinopathy" and "synucleopathic condition" refer to a condition characterized by abnormal profiles of oligomeric alpha-synuclein, which is an abnormal, aggregated form of alpha-synuclein. In certain embodiments, synucleinopathies manifest as clinically evident synucleopathic disease such as, for example, PD, Lewy body dementia, multiple system atrophy and some forms of Alzheimer's disease, as well as other rare neurodegenerative disorders such as various neuroaxonal dystrophies. Signs and, optionally, symptoms sufficient for a clinical diagnosis of a synucleinopathic disease are those generally sufficient for a person skilled in the art of diagnosing such conditions to make such a clinical diagnosis.

Parkinson's disease ("PD") is a progressive disorder of the central nervous system (CNS) with a prevalence of 1 % to 2% in the adult population over 60 years of age. PD is characterized by motor symptoms, including tremor, rigidity, postural instability and slowness of voluntary movement. The cause of the idiopathic form of the disease, which constitutes more than 90% of total PD cases, remains elusive, but is now considered to involve both environmental and genetic factors. Motor symptoms are clearly related to a progressive degeneration of dopamine-producing neurons in the substantia nigra. More recently, PD has become recognized one of a group of multi-system disorders, which mainly affect the basal ganglia (e.g., PD), or the cerebral cortex (e.g., Lewy body dementia), or the basal ganglia, brain stem and spinal cord (e.g., multiple system atrophy) and which are all linked by the presence of intracellular deposits (Lewy bodies) consisting mainly of a brain protein called alpha-synuclein. Accordingly, these disorders, along with Hallevorden-Spatz syndrome, neuronal axonal dystrophy, and traumatic brain injury have often been termed "Synucleinopathies".

Signs and symptoms of PD may include, for example, tremors at rest, rigidity, bradykinesia, postural instability and a festinating parkinsonian gate. One sign of PD is a positive response in these motor dysfunctions to carbidopa-levodopa.

Clinically recognized stages of Parkinson's disease include the following: Stage 1 - mild; Stage 2 - moderate; Stage 3 - middle stage; Stage 4-severe; Stage 5 - advanced.

At present, the diagnosis of PD mainly rests on the results of a physical examination that is often quantified by the use of the modified Hoehn and Yahr staging scale (Hoehn and Yahr, 1967, Neurology, 17:5, 427-442) and the Unified Parkinson's Disease Rating Scale (UPDRS). The differential diagnosis of PD vs. other forms of parkinsonism, e.g., progressive supranuclear palsy (PSP), can prove difficult and misdiagnosis can thus occur in up to 25% of patients. Indeed, PD generally remains undetected for years before the initial clinical diagnosis can be made. When this happens, the loss of dopamine neurons in the substantia nigra already exceeds 50% and may approach 70%. No blood test for PD or any related synucleinopathy has yet been validated. While imaging studies using positron emission tomography (PET) or MRI have been used in the diagnosis of PD by providing information about the location and extent of the neurodegenerative process, they confer little or no information about the pathogenesis of the observed degeneration and do not guide the selection of a particular synucleopathic-specific intervention.

Lewy body dementias (LBD) affect about 1.3 million people in the US. Symptoms include, for example, dementia, cognitive fluctuations, parkinsonism, sleep disturbances and hallucinations. It is the second most common form of dementia after Alzheimer's disease and usually develops after the age of 50. Like Parkinson's disease, LBD is characterized by abnormal deposits of alpha-synuclein in the brain.

Multiple system atrophy (MSA) is classified into two types, Parkinsonian type and cerebellar type. The parkinsonian type is characterized by, for example, parkinsonian symptoms of PD. The cerebellar type is characterized by, for example, impaired movement and coordination, dysarthria, visual disturbances and dysphagia. MSA symptoms reflect cell loss and gliosis or a proliferation of astrocytes in damaged areas of brain, especially the substantia nigra, striatum, inferior olivary nucleus, and cerebellum. Abnormal alpha-synuclein deposits are characteristic.

Diagnostic error rates for PD and other synucleinopathies can be relatively high, especially at their initial stages, a situation that could become important with the introduction of effective disease modifying therapies, such as neuroprotective therapies.

### 2. Alpha-synuclein

Alpha-synuclein is a protein found in the human brain. The human alpha-synuclein protein is made of 140 amino acids and is encoded by the SNCA gene (also called PARK1). (Alpha-synuclein: Gene ID: 6622; *Homo sapiens;* Cytogenetic Location: 4q22.1.)

As used herein, the term "alpha-synuclein" includes normal (unmodified) species, as well as modified species. Alpha-synuclein can exist in monomeric or aggregated forms. Alpha-synuclein monomers can aberrantly aggregate into oligomers, and oligomeric alpha-synuclein can aggregate into fibrils. Fibrils can further aggregate to form intracellular deposits called Lewy bodies. It is believed that monomeric alpha-synuclein and its various oligomers exist in equilibrium. Alpha-synuclein processing in brain can also produce other putatively abnormal species, such as alpha-synuclein phosphorylated at serine 129 ("p129 alpha-synuclein").

Alpha-synuclein is abundantly expressed in human central nervous system (CNS) and to a lesser extent in various other organs. In brain, alpha-synuclein is mainly found in neuronal terminals, especially in the cerebral cortex, hippocampus, substantia nigra and cerebellum, where it contributes to the regulation of neurotransmitter release. Under normal circumstances, this soluble monomeric protein tends to form a stably folded tetramer that resists aggregation. But, in certain pathological conditions, for unknown reasons, the alpha-synuclein abnormally beta pleats, misfolds, oligomerizes and aggregates to eventually form fibrils, a metabolic pathway capable of yielding highly cytotoxic intermediates.

As used herein, the term "monomeric alpha-synuclein" refers to a single, non-aggregated alpha-synuclein molecule, including any species thereof. As used herein, the term "oligomeric alpha-synuclein" refers to an aggregate comprising a plurality of alpha-synuclein protein molecules. This includes total oligomeric alpha-synuclein and forms or selected species thereof. Oligomeric alpha-synuclein includes forms having at least two monomeric units up to protofibril forms. This includes oligomeric forms having, e.g., between 2 and about 100 monomeric units, e.g., between 4 and 16 monomeric units or at least 2, 3, 4 or 5 dozen monomeric units. As used herein, the term "relatively low weight synuclein oligomer" refers to synuclein oligomers comprised of up to 30 monomeric units (30-mers). In certain embodiments, alpha-synuclein refers to the form or forms detected by the particular method of detection. For example, the forms can be those detectable with antibodies raised against particular monomeric or oligomeric forms of alpha-synuclein.

The neurotoxic potential of the aberrantly processed alpha-synuclein into oligomerized forms is now believed to contribute to the onset and subsequent progression of symptoms of the aforementioned pathological conditions, notably PD, Lewy body dementia, multiple system atrophy, and several other disorders. These are generally defined as a group of neurodegenerative disorders characterized in part by the intracellular accumulation of abnormal alpha-synuclein aggregates, some of which appear toxic and may contribute to the pathogenesis of the aforementioned disorders. Precisely how certain oligomerized forms of alpha-synuclein might cause neurodegeneration is not yet known, although a role for such factors as oxidative stress, mitochondrial injury, and pore formation has been suggested. Nevertheless, many now believe that processes leading to alpha-synuclein oligomerization and aggregation may be central to the cellular injury and destruction occurring in these disorders.

Some studies have shown that prefibrillar synuclein oligomers and protofibrils are especially prone to confer neurotoxicity (Loov et al., "α-Synuclein in Extracellular Vesicles: Functional Implications and Diagnostic Opportunities", M. Cell Mol Neurobiol. 2016 Apr;36(3):437-48. doi: 10.1007/s10571-015-0317-0.) Others suggest that lower order oligomeric synuclein species may be primarily responsible, and it remains hardly clear precisely which synuclein species, or which ensemble of species with differing beta-sheet arrangements, acting alone or in concert by a single or multiple pathologic mechanisms, is most neurotoxic in PD or in any related synucleinopathy (Wong et al., "α-synuclein toxicity in neurodegeneration: mechanism and therapeutic strategies", Nat Med. 2017 Feb 7;23(2):1-13. doi: 10.1038/nm.4269).

A portion of intracellular synuclein, along with certain of its metabolic products, is packaged within exosomal vesicles and released into the intracellular fluid in brain from where it passes into the cerebrospinal fluid (CSF) and peripheral blood circulation. Alpha-synuclein is a protein found in the human brain. The human alpha-synuclein protein is made of 140 amino acids and is encoded by the SNCA gene (also called PARK1). (Alpha-synuclein: Gene ID: 6622; *Homo sapiens;* Cytogenetic Location: 4q22.1.)

### B. Amyloidopathies

### 1. Conditions

As used herein, the term "amyloidopathy" refers to a condition characterized by accumulation of amyloid polymers in the brain. Amyloidopathies include, without limitation, Alzheimer's disease and certain other neurodegenerative disorders such as late stage PD. Alzheimer's Disease is the most prevalent form of dementia. It is characterized at an anatomical level by the accumulation of amyloid plaques made of aggregated forms of beta-amyloid, as well as neurofibrillary tangles. Symptomatically is characterized by progressive memory loss, cognitive decline and neurobehavioral changes. Alzheimer's is progressive and currently there is no known way to halt or reverse the disease.

### 2. Amyloid beta

Amyloid beta (also called amyloid-β, Aβ, A-beta and beta-amyloid) is a peptide fragment of amyloid precursor protein. Amyloid beta typically has between 36 and 43 amino acids. Amyloid beta aggregates to form soluble oligomers which may exist in several forms. It is believed that misfolded oligomers of amyloid beta can cause other amyloid beta molecules to assume a mis-folded oligomeric form. A-beta₁₋₄₂ has the amino acid sequence: DAEFRHDSGY EVHHQKLVFF AEDVGSNKGA IIGLMVGGVV IA [SEQ ID NO: 1].

In Alzheimer's disease, amyloid-β and tau proteins become oliogmerized and accumulate in brain tissue where they have appear to cause neuronal injury and loss; indeed, some aver that such soluble intermediates of aggregation, or oligomers, are the key species that mediate toxicity and underlie seeding and spreading in disease (The Amyloid-β Oligomer Hypothesis: Beginning of the Third Decade. Cline EN, Bicca MA, Viola KL, Klein WL. J Alzheimers Dis. 2018;64(s1):S567-S610; "Crucial role of protein oligomerization in the pathogenesis of Alzheimer's and Parkinson's diseases," Choi ML, Gandhi S. FEBS J. 2018 Jun 20.) Amyloid β oligomers are crucial for the onset and progression of AD and represent a popular drug target, being presumably the most direct biomarker. Tau protein may also become abnormally hyperphosphorylated.

Methods in current use to quantify monomeric and oligomeric forms of A-beta include enzyme linked immunosorbent assays (ELISA), methods for single oligomer detection, and others, which are mainly biosensor-based methods. ("Methods for the Specific Detection and Quantitation of Amyloid-β Oligomers in Cerebrospinal Fluid", Schuster J, Funke SA. J Alzheimers Dis. 2016 May 7;53(1):53-67.)

The surface-based fluorescence intensity distribution analysis (sFIDA) features both highly specific and sensitive oligomer quantitation as well as total insensitivity towards monomers ("Advancements of the sFIDA method for oligomer-based diagnostics of neurodegenerative diseases", Kulawik A. et al., FEBS Lett. 2018 Feb;592(4):516-534).

### C. Tauopathies

### 1. Conditions

As used herein, the term "tauopathy" refers to a condition characterized by accumulation of and aggregation of in association with neurodegeneration. Tauopathies include, without limitation, Alzheimer's disease ("AD"), progressive supranuclear palsy, corticobasal degeneration, frontotemporal dementia with parkinsonism-linked to chromosome 17, and Pick disease.

AD is also characterized by a second pathological hallmark, the neurofibrillary tangle (NFT). NFTs are anatomically associated with neuronal loss, linking the process of NFT formation to neuronal injury and brain dysfunction. The main component of the NFT is a hyperphosphorylated form of tau, a microtubule-associated protein. During NFT formation, tau forms a variety of different aggregation species, including tau oligomers. Increasing evidence indicates that tau oligomer formation precedes the appearance of neurofibrillary tangles and contributes importantly to neuronal loss. (J Alzheimers Dis. 2013;37(3):565-8 "Tauopathies and tau oligomers", Takashima A.)

Nonfibrillar, soluble multimers appear to be more toxic than neurofibrillary tangles made up of filamentous tau.

In frontotemporal lobe dementia, full-length TAR DNA Binding Protein ("TDP-43") forms toxic amyloid oligomers that accumulate in frontal brain regions. TDP-43 proteinopathies, which also include amyotrophic lateral sclerosis (ALS), are characterized by inclusion bodies formed by polyubiquitinated and hyperphosphorylated full-length and truncated TDP-43. The recombinant full-length human TDP-43 forms structurally stable, spherical oligomers that share common epitopes with an anti-amyloid oligomer-specific antibody. The TDP-43 oligomers have been found to be neurotoxic both in vitro and in vivo. (Nat Commun. 2014 Sep 12;5:4824. Full-length TDP-43 forms toxic amyloid oligomers that are present in frontotemporal lobar dementia-TDP patients). Determination of the presence and abundance of TDP-43 oligomers can be accomplished using a specific TDP-43 amyloid oligomer antibody called TDP-O among different subtypes of FTLD-TDP ("Detection of TDP-43 oligomers in frontotemporal lobar degeneration-TDP", Kao PF, Ann Neurol. 2015 Aug;78(2):211-21.)

### 2. Tau

Tau is a phosphoprotein with 79 potential Serine (Ser) and Threonine (Thr) phosphorylation sites on the longest tau isoform. Tau exists in six isoforms, distinguished by their number of binding domains. Three isoforms have three binding domains and the other three have four binding domains. The isoforms result from alternative splicing in exons 2, 3, and 10 of the tau gene. Tau is encoded by the MAPT gene, which has 11 exons. Haplogroup H1 appears to be associated with increased probability of certain dementias, such as Alzheimer's disease.

Various tau oligomeric species, including those ranging from 6- to 18-mers, have been implicated in the neurotoxic process associated with tauopathic brain disorders and measured by western blot and other techniques including single molecule fluorescence. (See, e.g., Kjaergaard M., et al., "Oligomer Diversity during the Aggregation of the Repeat Region of Tau" ACS Chem Neurosci. 2018 Jul 17; Ghag G et al., "Soluble tau aggregates, not large fibrils, are the toxic species that display seeding and cross-seeding behavior", Protein Sci. 2018 Aug 20. doi: 10.1002/pro.3499; and Comerota MM et al., "Near Infrared Light Treatment Reduces Synaptic Levels of Toxic Tau Oligomers in Two Transgenic Mouse Models of Human Tauopathies", Mol Neurobiol. 2018 Aug 17.)

Methods to measure oligomeric tau species include immunoassay. Tau can be isolated by a common expression followed by chromatography, such as affinity, size-exclusion, and anion-exchange chromatography. This form can be used to immunize animals to generate antibodies. Aggregation of tau can be induced using arachidonic acid. Oligomers can be purified by centrifugation over a sucrose step gradient. Oligomeric forms of tau also can be used to immunize animals and generate antibodies. A sandwich enzyme-linked immunosorbent assay that utilizes the tau oligomer-specific TOC1 antibody can be used to detect oligomeric tau. The tau oligomer complex 1 (TOC1) antibody specifically identifies oligomeric tau species, in the tris insoluble, sarkosyl soluble fraction. (Shirafuji N., et al, "Homocysteine Increases Tau Phosphorylation, Truncation and Oligomerization", Int J Mol Sci. 2018 Mar 17;19(3).) (See, e.g., Methods Cell Biol. 2017;141:45-64. doi: 10.1016/bs.mcb.2017.06.005. Epub 2017 Jul 14. Production of recombinant tau oligomers in vitro. Combs B1, Tiernan CT1, Hamel C1, Kanaan NM.)

### D. Huntington's Disease

### 1. Huntington's Disease

Huntington's disease is an inherited disease caused by an autosomal dominant mutation in the huntingtin gene. The mutation is characterized by duplication of CAG triplets. It is characterized by progressive neurodegeneration. Symptoms include movement disorders, such as involuntary movements, impaired gait and difficulty with swallowing and speech. It is also characterized by a progressive cognitive decline.

### 2. Huntingtin Protein

Huntington protein is encoded by the Huntington gene also called HTT or HD. The normal Huntington protein has about 3144 amino acids. The protein is normally about 300 KdA.

In Huntington's disease (HD), cleavage of the full-length mutant huntingtin (mhtt) protein into smaller, soluble aggregation-prone mhtt fragments appears to be a key process in the pathophysiology of this disorder. Indeed, aggregation and cytotoxicity of mutant proteins containing an expanded number of polyglutamine (polyQ) repeats is a hallmark of several diseases, in addition to HD. Within cells, mutant Huntingtin (mHtt) and other polyglutamine expansion mutant proteins exist as monomers, soluble oligomers, and insoluble inclusion bodies. (J Huntingtons Dis. 2012;1(1):119-32. Detection of Mutant Huntingtin Aggregation Conformers and Modulation of SDS-Soluble Fibrillar Oligomers by Small Molecules. Sontag EM, et al., Brain Sci. 2014 Mar 3;4(1):91-122. Monomeric, oligomeric and polymeric proteins in Huntington disease and other diseases of polyglutamine expansion. Hoffner G. et al.) In certain embodiments, oligomers are 2-10 nm in height with an aspect ratio (longest distance across to shortest distance across) less than 2.5, indicating a globular structure.

### II. Detection and Measurement of Monomers and Oligomers

### A. Biological Samples

As used herein, the term "sample" refers to a composition comprising an analyte. A sample can be a raw sample, in which the analyte is mixed with other materials in its native form (e.g., a source material), a fractionated sample, in which an analyte is at least partially enriched, or a purified sample in which the analyte is at least substantially pure. As used herein, the term "biological sample" refers to a sample comprising biological material including, e.g., polypeptides, polynucleotides, polysaccharides, lipids and higher order levels of these materials such as, exosomes cells, tissues or organs.

Oligomeric and monomeric forms of neurodegenerative proteins, such as alpha-synuclein, amyloid beta, tau and huntingtin, can be detected in exosomes from bodily fluid samples from the subject. More particularly, isolates of CNS-derived exosomes are a preferred subset of exosomes for the detection and analysis of synucleopathic conditions. In particular, proteins from internal compartments of an exosome are useful.

Exosomes can be isolated from a variety of biological samples from a subject. The biological sample is a bodily fluid. Bodily fluid sources of exosomes include, for example, blood (e.g., whole blood or a fraction thereof such as serum or plasma, e.g., peripheral venous blood), cerebrospinal fluid, saliva, milk and urine, or fractions thereof. In the claimed invention, the biological sample is a blood sample. The use of venous blood as a source of exosomes is a preferred sample for a diagnostic test destined for use in both adults and children due to the safety, acceptability and convenience of routine venipuncture in medical settings.

### B. Methods of Determining Amounts of Oligomeric and

### Monomeric Polypeptides

Monomeric and oligomeric forms of proteins can be detected by any methods known in the art including, without limitation, immunoassay (e.g., ELISA), mass spectrometry, size exclusion chromatography, Western blot and fluorescence-based methods (e.g., fluorescence spectroscopy or FRET) and proximity ligation assay.

### 1. Alpha-synuclein

Amounts of monomeric alpha-synuclein and oligomeric alpha-synuclein can be determined individually. Alternatively, total alpha-synuclein in the sample can be measured with either of monomeric alpha-synuclein or oligomeric alpha-synuclein and the amount of the other species can be determined based on the difference.

Monomeric, oligomeric and total alpha-synuclein can be detected by, for example, immunoassay (e.g., ELISA or Western blot), mass spectrometry or size exclusion chromatography. Antibodies against alpha-synuclein are commercially available from, for example, Abcam (Cambridge, MA), ThermoFisher (Waltham, MA) and Santa Cruz Biotechnology (Dallas, TX).

The following references described methods of measuring total alpha-synuclein content. Mollenhauer et al. (Movement Disorders, 32:8 p. 1117 (2017)) describes methods of measuring total alpha-synuclein from bodily fluids. Loov et al. (Cell Mol. Neurobiol., 36:437-448 (2016)) describes use of antibodies to isolate L1 CAM positive exosomes from plasma. Abd-Elhadi et al. (Anal Bioanal Chem. (2016) Nov;408(27):7669-72016) describes methods of determining total alpha-synuclein levels in human blood cells, CSF, and saliva determined by a lipid-ELISA.

Total alpha-synuclein can be detected in an ELISA using, for example, an anti-human α-syn monoclonal antibody 211 (Santa Cruz Biotechnology, USA) for capture and anti-human α-syn polyclonal antibody FL-140 (Santa Cruz Biotechnology, USA) for detection through a horseradish peroxidase (HRP)-linked chemiluminescence assay. Such an approach avoids detection of monomeric α-synuclein, but does not distinguish between the different multimeric forms.

Monomeric and oligomeric forms of alpha-synuclein can be detected by, for example, immunoassays using antibodies specific for the forms. See, e.g., Williams et al. ("Oligomeric alpha-synuclein and β-amyloid variants as potential biomarkers for Parkinson's and Alzheimer's diseases", Eur J Neurosci. (2016) Jan;43(1):3-16) and Majbour et al. ("Oligomeric and phosphorylated alpha-synuclein as potential CSF biomarkers for Parkinson's disease", Molecular Neurodegeneration (2016) 11:7). El-Agnaf O. et al, (FASEB J. 2016;20:419-425) described detection of oligomeric forms of alpha-synuclein protein in human plasma as a potential biomarker for PD.

Antibodies against alpha-synuclein monomers and oligomers can be produced by immunizing animals with alpha-synuclein monomers or oligomers. (See, e.g., U.S. Publications 2016/0199522 (Lannfelt et al.), 2012/0191652 (El-Agnaf). Alpha-synuclein oligomers can be prepared by the method of El Agnaf (U.S. 2014/0241987), in which freshly prepared α-synuclein solution was mixed with dopamine at 1:7 molar ratio (α-synuclein:dopamine) and incubated at 37° C. Antibodies against different oligomeric forms of alpha-synuclein are also described in Emadi et al. ("Isolation of a Human Single Chain Antibody Fragment Against Oligomeric α-Synuclein that Inhibits Aggregation and Prevents α-Synuclein-induced Toxicity", J Mol Biol. 2007; 368:1132-1144. [PubMed: 17391701]) (dimers and tetramers) and Emadi et al. ("Detecting Morphologically Distinct Oligomeric Forms of α-Synuclein", J Biol Chem. 2009; 284:11048-11058. [PubMed: 19141614]) (trimers and hexamers). Protofibril-binding antibodies are described in, for example, U.S. 2013/0309251 (Nordstrom et al.).

Monomeric alpha-synuclein and can be distinguished from polymeric alpha-synuclein by immunoassay using antibodies that are uniquely recognized by oligomeric forms of synuclein. Another method involves detection of mass differences, e.g., using mass spectrometry. Fluorescent methods can be used. (See, e.g., Sangeeta Nath, et al., "Early Aggregation Steps in α-Synuclein as Measured by FCS and FRET: Evidence for a Contagious Conformational Change" Biophys J. 2010 Apr 7; 98(7): 1302-1311, doi: 10.1016/j.bpj.2009.12.4290; and Laura Tosatto et al., "Single-molecule FRET studies on alpha-synuclein oligomerization of Parkinson's disease genetically related mutants", Scientific Reports 5, December 2015.) Another method involves measuring total alpha synuclein, followed by proteinase K digestion of non-pathological alpha synuclein and detection of remaining alpha synuclein. Another method involves an alpha synuclein proximity ligation assay. Protein ligation assay probes are generated from antibodies raised against the protein(s) of interest, one for each of the proteins involved in the putative interaction, which are conjugated to short oligonucleotides. If the probes bind interacting proteins, the oligonucleotides are sufficiently close to prime an amplification reaction, which can be detected by tagged oligonucleotides and observed as punctate signal, with each punctum representing an interaction. (Roberts RF et al., "Direct visualization of alpha-synuclein oligomers reveals previously undetected pathology in Parkinson's disease brain. Brain", 2015;138:1642-1657. doi: 10.1093/brain/awv040, and Nora Bengoa-Vergniory et al., "Alpha-synuclein oligomers: a new hope", Acta Neuropathol. 2017; 134(6): 819-838).

The relative amount of oligomeric form of alpha-synuclein to monomers can be expressed as a ratio.

Quantity or amount can be expressed as a signal output from an assay or as an absolute amount after conversion, for example from a standard curve, e.g., in terms of mass per volume.

Alpha-synuclein species in the samples can be further stratified. For example, oligomers species can be divided into lower order oligomers, e.g., 2 to 24 monomeric units, higher order oligomers, e.g., 24 to 100 monomeric units, or protofibrils, etc.

### 2. Amyloid beta

Oligomers and monomers can be distinguished using an enzyme-linked immunosorbent assay (ELISA). This assay resembles a sandwich ELISA. The Aβ monomer contains one epitope, while oligomers contain a plurality these epitopes. Hence, if epitope-overlapping antibodies toward the above unique epitope were used for capturing and detecting antibodies, binding to a specific and unique epitope would generate competition between these two antibodies. In other words, the monomer would be occupied by the capturing or detection antibody but not by both. ("Oligomeric forms of amyloid-β protein in plasma as a potential blood-based biomarker for Alzheimer's disease", Wang MJ et al. Alzheimers Res Ther. 2017 Dec 15;9(1):98. "Potential fluid biomarkers for pathological brain changes in Alzheimer's disease: Implication for the screening of cognitive frailty", Ruan Q et al., Mol Med Rep. 2016 Oct;14(4):3184-98. "Methods for the Specific Detection and Quantitation of Amyloid-β Oligomers in Cerebrospinal Fluid," Schuster J, Funke SA. J Alzheimers Dis. 2016 May 7;53(1):53-67).

Oligomeric forms of amyloid beta for detection include, e.g. 4-24 mers of amyloid beta.

### 3. Tau

Tau oligomers in biological fluids, e.g., CSF, can be measured by ELISA and Western blot analysis using anti-tau oligomer antibodies. (Sengupta U, et al., "Tau oligomers in cerebrospinal fluid in Alzheimer's disease", Ann Clin Transl Neurol. 2017 Apr; 4(4): 226-235.

Oligomers of tau for detection include, e.g., low molecular weight oligomers, e.g., no more than 20-mers, e.g., 3-18 mers. The presence of soluble oligomers in the cerebral spinal fluid can be detected with monoclonal anti-oligomer antibodies with Western blot and Sandwich enzyme-linked immunosorbent assay (sELISA). David, MA et al., "Detection of protein aggregates in brain and cerebrospinal fluid derived from multiple sclerosis patients", Front Neurol. 2014 Dec 2;5:251. Oligomeric forms of tau include hyperphosphorylated forms of oligomeric tau.

### 4. Huntingtin

Recent quantification studies have made use of TR-FRET-based immunoassays. One detection method that combines size exclusion chromatography (SEC) and time-resolved fluorescence resonance energy transfer (TR-FRET) allows the resolution and definition of the formation, and aggregation of native soluble mhtt species and insoluble aggregates in brain. "Fragments of HdhQ150 mutant huntingtin form a soluble oligomer pool that declines with aggregate deposition upon aging", Marcellin D. et al., PLoS One. 2012;7(9):e44457.

A variety of published techniques have been used to assay oligomeric huntingtin species including, e.g., Agarose Gel Electrophoresis (AGE) analysis (under either native or mildly denaturing, 0.1% SDS conditions or Blue-Native PAGE under native conditions) which provides a number of immunoreactive oligomers; Anti-huntingtin antibodies differentially recognize specific huntingtin oligomers.

A one-step TR-FRET-based immunoassay has been developed to quantify soluble and aggregated mHtt in cell and tissue homogenates (TR-FRET-based duplex immunoassay reveals an inverse correlation of soluble and aggregated mutant huntingtin in Huntington's disease. Baldo B, et al., chem Biol. 2012 Feb 24;19(2):264-75).

Time-resolved Förster energy transfer (TR-FRET)-based assays represent high-throughput, homogeneous, sensitive immunoassays widely employed for the quantification of proteins of interest. TR-FRET is extremely sensitive to small distances and can therefore provide conformational information based on detection of exposure and relative position of epitopes present on the target protein as recognized by selective antibodies. We have previously reported TR-FRET assays to quantify HTT proteins based on the use of antibodies specific for different amino-terminal HTT epitopes (Fodale, V. et al., "Polyglutamine- and temperature-dependent conformational rigidity in mutant huntingtin revealed by immunoassays and circular dichroism spectroscopy", PLoS One. 2014 Dec 2;9(12):e112262. doi: 10.1371/journal.pone.0112262. eCollection 2014.

### C. Isolation of Exosomes

Exosomes are extracellular vesicles that are thought to be released from cells upon fusion of an intermediate endocytic compartment, the multivesicular body (MVB), with the plasma membrane. The vesicles released in this process are referred to as exosomes. It is believed that exosomes contribute to the spread of toxic synuclein species between CNS neurons and into the CSF and other bodily fluids. Exosomes are typically in the range of about 20 nm to about 100 nm.

Many methods of isolating exosomes are known in the art. These include, for example, immunoaffinity capture methods, size-based isolation methods, differential ultracentrifugation, exosome precipitation, and microfluidic-based isolation techniques. Loov C. et al. Cell Neurobiol. 2016 (supra).

Immunoaffinity capture methods use antibodies attached to an extraction moiety to bind exosomes and separates them from other materials in the sample. A solid support can be, for example, a magnetically attractable microparticle. Latex immunobeads can be used.

In particular, this method is useful for isolating CNS-derived exosomes using brain-specific biomarkers (e.g., neural and glial markers) one such marker is L1CAM. Another marker is KCAM. Other relatively brain-specific proteins can also serve in this capacity. CNS-derived exosomes are characterized by protein markers associated with the brain, including, for example, KCAM, L1CAM and NCAM. (See, e.g., US 2017/0014450, US 2017/0102397, US 9,958,460). CNS derived exosomes can be isolated using affinity capture methods. Such methods include, for example, paramagnetic beads attached to antibodies against specific markers such as L1 CAM. (See, e.g., Shi et al., "Plasma exosomal α-alpha-synuclein is likely CNS-derived and increased in Parkinson's disease", Acta Neuropathol. 2014 November; 128(5): 639-650.)

Qiagen describes its exoEasy Maxi Kit as using membrane affinity spin columns to efficiently isolate exosomes and other extracellular vesicles from serum, plasma, cell culture supernatant and other biological fluids.

Size-based isolation methods include, for example, size exclusion chromatography and ultrafiltration. In size exclusion chromatography a porous stationary phase is used to separate exosomes based on size. In ultrafiltration, a porous membrane filter is used two separate exosomes based on their size or weight.

Differential ultracentrifugation involves a series of centrifugation cycles of different centrifugal force and duration to isolate exosomes based on their density and size differences from other components in a sample. The centrifugal force can be, for example, from ~100,000 to 120,000 × g. Protease inhibitors can be used to prevent protein degradation. A prior cleanup step can be used to remove other large material from the sample.

Exosomes can be isolated from solutions of biological materials by altering their solubility or dispersibility. For example, addition of polymers such as polyethylene glycol (PEG), e.g., with a molecular weight of 8000 Da, can be used to precipitate exosomes from solution.

Microfluidics-based methods can be used to isolate exosomes. These includes, for example, acoustic, electrophoretic and electromagnetic methods. For example, an acoustic nanofilter uses ultrasound standing waves to separate exosomes in a sample according to their size and density.

Other methods of isolating CNS-derived exosomes are described in, for example, Kanninnen, KM et al., "Exosomes as new diagnostic tools in CNS diseases", Biochimica et Biophysica Acta, 1862 (2016) 403-410.

Many proteins, such as alpha synuclein, linked to the pathogenesis of human neurodegenerative disease, are generated outside the CNS as well as within the brain and can become attached to the external surface of exosomes that pass through the blood brain barrier into the peripheral circulation. Accordingly, in certain embodiments of the methods disclosed herein, an exosomal fraction is treated to remove molecules bound to the exosomal surface. This can be done, for example, by stringent washing procedures, such as with a Phosphate Buffer Solution (PBS). After such processing, the contents of the exosome can be processed for the assay.

### III. Determining Diagnosis, Stage, Progression, Prognosis and Risk of Developing of Neurodegenerative Conditions

Biomarker profiles comprising amounts of oligomeric and, optionally, monomeric forms of a neurodegenerative protein biomarker (e.g., amounts of species of oligomeric and, optionally, monomeric alpha-synuclein; oligomeric and, optionally, monomeric amyloid beta, oligomeric and, optionally hyperphosphorylated and, optionally, monomeric tau; and oligomeric and, optionally, monomeric huntingtin) in a biological sample, and a change in the profiles over time, indicate presence, severity and direction of neurogenerative conditions of the neurodegenerative type. In particular, abnormal ratios, e.g., elevated amounts, of the protein biomarker disclosed herein indicate a process of neurodegeneration. This process, unchecked, can lead to manifest symptoms in synucleopathic conditions. Accordingly, provided herein are methods of ascertaining in a subject (e.g., in either symptomatic or asymptomatic individuals) a diagnosis, stage, progression, rate, prognosis, drug responsiveness and risk of developing a neurodegenerative condition characterized by the abnormal amounts of aggregated protein, e.g., alpha-synuclein, amyloid beta, tau or huntingtin (each, referred to herein as a "neuropathic state", e.g. "synucleinopathic state", "amyloidopathic state", "tauopathic state", "Huntington's state").

As used herein, the term "diagnosis" refers to a classification of an individual as having or not having a particular pathogenic condition, including, e.g., the stage of that condition.

As used herein, the term "clinically similar but etiologically different" refers to conditions that share clinical signs and/or symptoms but which arise from different biological causes.

As used herein, the term "stage" refers to the relative degree of severity of a condition, for example, suspected disease, an early stage, a middle stage or an advanced stage. Staging can be used to group patients based on etiology, pathophysiology, severity, etc.

As used herein, the term "progression" refers to a change, or lack thereof, in stage or severity of a condition over time. This includes an increase, a decrease or stasis in severity of the condition. In certain embodiments, rates of progression, that is, change over time, are measured.

As used herein, the term "prognosis" refers to the predicted course, e.g., the likelihood of progression, of the condition. For example, a prognosis may include a prediction that severity of the condition is likely to increase, decrease or remain the same at some future point in time. In the context of the present disclosure, prognosis can refer to the likelihood that an individual: (1) will develop a neurodegenerative condition, (2) will progress from one stage to another, more advanced, stage of the condition, (3) will exhibit a decrease in severity of the condition, (4) will exhibit functional decline at a certain rate, (5) will survive with a condition for a certain period of time (e.g., survival rate) or (6) will have recurrence of the condition. The condition can be a synucleopathic condition (e.g., PD, Lewy body dementia, multiple system atrophy or some related synucleinopathy), an amyloidopathic condition (e.g., Alzheimer's disease), a tauopathic condition (e.g., Alzheimer's disease), and Huntington's disease. These terms are not intended to be absolute, as will be appreciated by any one of skill in the field of medical diagnostics.

As used herein, the term "risk of developing" refers to a probability that an individual who is asymptomatic or preclinical will develop to a definitive diagnosis of disease. Determining probability includes both precise and relative probabilities such as "more likely than not", "highly likely", "unlikely", or a percent chance, e.g., "90%". Risk can be compared with the general population or with a population matched with the subject based on any of age, sex, genetic risk, and environmental risk factors. In such a case, a subject can be determined to be at increased or decreased risk compared with other members of the population.

In general, increasing relative amounts of oligomeric to monomeric neurodegenerative proteins, such as alpha-synuclein, beta amyloid, tau and huntingtin, are correlated with neurodegenerative processes, presence of disease, more advanced stage of disease, progression to a more serious stage, worse prognosis, increased risk of developing disease or the ineffectiveness of an experimental therapeutic intervention. In certain embodiments, it is preferred to measure alpha-synuclein from CNS-derived exosomes in peripheral blood. For example, an increase in relative amounts of oligomeric to monomeric forms of at least 10%, at least 20%, at least 50%, at least 100% at least 250%, at least 500% or at least 1000% compared to normal indicates an abnormal condition, such as presence of disease.

### IV. Modeling Profiles of Species of Neurodegenerative Proteins to Infer Diagnosis, Stage, Progression, Prognosis and Risk of Developing of Neurodegenerative Conditions

Determining diagnosis, stage, progression, prognosis and risk of a neurodegenerative condition are processes of classifying a subject into different conditions or different classes or conditions within a state, such as disease/health (diagnosis), stage I/stage II/stage III (stage), likely to remiss/likely to progress (prognosis) or assigning a score on a range. Methods of classification using biomarker profiles can involve identifying profiles that are characteristic of various states and correlating a profile from a subject with class or state. Identifying such profiles can involve analysis of biomarker profiles from subjects belonging to different states and discerning patterns or differences between the profiles. Analysis can be done by visual examination of the profiles or by statistical analysis.

### A. Statistical Analysis

Typically, analysis involves statistical analysis of a sufficiently large number of samples to provide statistically meaningful results. Any statistical method known in the art can be used for this purpose. Such methods, or tools, include, without limitation, correlational, Pearson correlation, Spearman correlation, chisquare, comparison of means (e.g., paired T-test, independent T-test, ANOVA) regression analysis (e.g., simple regression, multiple regression, linear regression, non-linear regression, logistic regression, polynomial regression, stepwise regression, ridge regression, lasso regression, elasticnet regression) or non-parametric analysis (e.g., Wilcoxon rank-sum test, Wilcoxon sign-rank test, sign test). Such tools are included in commercially available statistical packages such as MATLAB, JMP Statistical Software and SAS. Such methods produce models or classifiers which one can use to classify a particular biomarker profile into a particular state.

Statistical analysis can be operator implemented or implemented by machine learning.

### B. Machine Learning

In certain embodiments statistical analysis is enhanced through the use of machine learning tools. Such tools employ learning algorithms, in which the relevant variable or variables are measured in the different possible states, and patterns differentiating the states are determined and used to classify a test subject. Accordingly, any classification method of this disclosure can be developed by comparing measurements of one or more variables in subjects belonging to the various conditions within a particular synucleinopathic state. This includes, for example, determining a biomarker profile comprising amounts of one or more forms of oligomeric alpha-synuclein and, optionally, monomeric alpha-synuclein in subjects with various diagnoses or at various stages at various times to allow prediction of diagnosis, stage, progression, prognosis, drug responsiveness or risk. Other variables can be included as well, such as family history, lifestyle, exposure to chemicals, various phenotypic traits, etc.

### 1. Training Dataset

A training dataset is a dataset typically comprising a vector of values for each of a plurality of features for each of a plurality of subjects (more generally referred to as objects). One of the features can be a classification of the subject, for example, a diagnosis or a measure of a degree on a scale. This can be used in supervised learning methods. Other features can be, for example, measured amounts of each of a plurality of different forms of a neurodegenerative protein. The different forms will include a plurality of different species including, for example, one or a plurality of oligomeric forms and, optionally one or a plurality of monomeric forms. Typically, the features will include a plurality of different oligomeric forms and, optionally, one or more monomeric forms. So, for example, a vector for an individual subject can include a diagnosis of a neurodegenerative condition (e.g., diagnosed as having or not having Parkinson's Disease) and measurements of a plurality of forms selected from monomeric alpha synuclein, dimeric alpha synuclein, trimeric alpha synuclein, tetrameric alpha synuclein, ... 28-mer alpha synuclein, 29-mer alpha synuclein and 30-mer alpha synuclein. In other embodiments the forms are collections of species, such as relatively low molecular weight alpha-synuclein species. In certain embodiments, the training dataset used to generate the classifier comprises data from at least 100, at least 200, or at least 400 different subjects. The ratio of subjects classified has having versus not having the condition can be at least 2:1, at least 1:1, or at least 1:2. Alternatively, subjects pre-classified as having the condition can comprise no more than 66%, no more than 50%, no more than 33% or no more than 20% of subjects.

### 2. Learning Algorithms

Learning algorithms, also referred to as machine learning algorithms, are computer-executed algorithms that automate analytical model building, e.g., for clustering, classification or profile recognition. Learning algorithms perform analyses on training datasets provided to the algorithm.

Learning algorithms output a model, also referred to as a classifier, classification algorithm or diagnostic algorithm. Models receive, as input, test data and produce, as output, an inference or a classification of the input data as belonging to one or another class, cluster group or position on a scale, such as diagnosis, stage, prognosis, disease progression, responsiveness to a drug, etc.

A variety of machine learning algorithms can be used to infer a condition or state of a subject. Machine learning algorithms may be supervised or unsupervised. Learning algorithms include, for example, artificial neural networks (e.g., back propagation networks), discriminant analyses (e.g., Bayesian classifier or Fischer analysis), support vector machines, decision trees (e.g., recursive partitioning processes such as CART - classification and regression trees), random forests, linear classifiers (e.g., multiple linear regression (MLR), partial least squares (PLS) regression and principal components regression (PCR)), hierarchical clustering and cluster analysis. The learning algorithm will generate a model or classifier that can be used to make an inference, e.g., an inference about a disease state of a subject.

### 3. Validation

A model may be subsequently validation using a validation dataset. Validation datasets typically include data on the same features as the training dataset. The model is executed on the training dataset and the number of true positives, true negatives, false positives and false negatives is determined, as a measure of performance of the model.

The model can then be tested on a validation dataset to determine its usefulness. Typically, a learning algorithm will generate a plurality of models. In certain embodiments, models can be validated based on fidelity to standard clinical measures used to diagnose the condition under consideration. One or more of these can be selected based on its performance characteristics.

### C. Computers

Classification of a subject's condition based on any of the states described herein can be performed by a programmable digital computer. The computer can include tangible memory that receives and, optionally stores at least measurements of one or a plurality of oligomeric forms and, optionally, monomeric forms of the protein biomarker (e.g., species of oligomeric and, optionally, monomeric alpha-synuclein; oligomeric and, optionally monomeric amyloid beta, oligomeric and, optionally, monomeric tau; and oligomeric and, optionally, monomeric huntingtin), in a subject and a processor that processes this data by the execution of code embodying a classification algorithm. The classification algorithm can be the result of operator-implemented or machine learning-implemented statistical analysis.

A system comprises a first computer as described in communication with a communications network configured to transmit data to the computer and/or transmit results of a test, such as a classification as described herein to a remote computer. The communications network can utilize, for example, a high-speed transmission network including, without limitation, Digital Subscriber Line (DSL), Cable Modem, Fiber, Wireless, Satellite and, Broadband over Powerlines (BPL). The system can further comprise a remote computer connected through the communications network to the first computer.

### D. Model Execution and Making an Inference

The model selected can either result from operator executed statistical analysis or machine learning. In any case, the model can be used to make inferences (e.g., predictions) about a test subject. A biomarker profile, for example in the form of a test dataset, e.g., comprising a vector, containing values of features used by the model, can be generated from a sample taken from the test subject. The test dataset can include all of the same features used in the training dataset, or a subset of these features. The model is then applied to or executed on the test dataset. Correlating a neurodegenerative protein profile with a condition, disease state, a prognosis, a risk of progression, a likelihood of drug response, etc. is a form of executing a model. Correlating can be performed by a person or by a machine. The choice may depend on the complexity of the operation of correlating. This produces an inference, e.g., a classification of a subject as belonging to a class or a cluster group (such as a diagnosis), or a place on a scale (such as likelihood of responding to a therapeutic intervention).

In certain embodiments the classifier will include a plurality of oligomeric protein forms and, typically, but not necessarily, one or more monomeric forms of the neurodegenerative protein. The classifier may or may not be a linear model, e.g., of the form AX+BY+CZ = N, wherein A, B and C are measured amounts of forms X, Y and Z. The classifier may require, for example, support vector machine analysis. For example, the inference model may perform a pattern recognition in which a biomarker profile lies on a scale between normal and abnormal, with various profiles tending more toward normal or toward abnormal. Thus, the classifier may indicate a confidence level that the profile is normal or abnormal.

The classifier or model may generate, from the one or are plurality of forms measured, a single diagnostic number which functions as the model. Classifying a neuropathological state, e.g., synucleopathic state (e.g., diagnosis, stage, progression, prognosis and risk) can involve determining whether the diagnostic number is above or below a threshold ("diagnostic level"). For example, the diagnostic number can be a relative amount of oligomeric neurodegenerative protein (e.g., alpha-synuclein) to monomeric neurodegenerative protein (e.g., alpha-synuclein) (including measuring specific species or phosphorylated forms of each). That threshold can be determined, for example, based on a certain deviation of the diagnostic number above normal individuals who are free of any sign of a neurodegenerative, e.g., synucleopathic, condition. A measure of central tendency, such as mean, median or mode, of diagnostic numbers can be determined in a statistically significant number of normal and abnormal individuals. A cutoff above normal amounts can be selected as a diagnostic level of a neurodegenerative, e.g., synucleinopathic, condition. That number can be, for example, a certain degree of deviation from the measure of central tendency, such as variance or standard deviation. In one embodiment the measure of deviation is a Z score or number of standard deviations from the normal average. In certain embodiments, a quantity of oligomeric alpha-synuclein to monomeric alpha-synuclein greater than 1.5:1, 2:1, 5:1, or 10:1 indicates the presence or increased risk of manifesting a neurodegenerative, e.g., synucleopathic condition.

The model can be selected to provide a desired level of sensitivity, specificity or positive predictive power. For example, the diagnostic level can provide a sensitivity of at least any of 80%, 90%, 95% or 98% and/or a specificity of at least any of 80%, 90%, 95% or 98%, and/or a positive predictive value of at least any of 80%, 90%, 95% or 98%. The sensitivity of a test is the percentage of actual positives that test positive. The specificity of a test is the percentage of actual negatives that test negative. The positive predictive value of a test is the probability that a subject that tests positive is an actual positive.

### V. Development of Therapeutic Interventions to Treat Neurodegenerative Conditions

In another aspect, provided herein are methods to enable the practical development of therapeutic interventions for neurodegenerative conditions, e.g., synucleopathic conditions, amyloidopathic conditions, tauopathic conditions, and Huntington's disease. The methods involve, among other things, selecting subjects for clinical trials and determining effectiveness of the therapeutic intervention in a set of subjects.

Methods comprising monitoring the biomarker profiles of neurodegenerative proteins (e.g., species of oligomeric and, optionally, monomeric alpha-synuclein; oligomeric and, optionally, monomeric amyloid beta, oligomeric and, optionally, monomeric tau; and oligomeric and, optionally, monomeric huntingtin) are useful to determine whether an experimental therapeutic intervention is effective in preventing clinical onset or inhibiting subsequent progression of a synucleinopathy, or whether a subject should be entered into a clinical trial to test the efficacy of a drug candidate to treat such conditions. Biomarker profiles or changes in biomarker profiles of the neurodegenerative protein (e.g., relative amounts or rates of change in relative amounts of oligomeric and monomeric forms of the protein biomarker (e.g., species of oligomeric and monomeric alpha-synuclein; oligomeric and monomeric amyloid beta, oligomeric and monomeric tau; and oligomeric and monomeric huntingtin) enable the direct determination of treatment effects on the condition, including, e.g., basic disease process.

### A. Subject Enrollment

Clinical trials involve enrollment of subjects for testing the efficacy and safety of a potential therapeutic intervention, such as a pharmaceutical. Typically, subjects are selected to have different conditions of a state, e.g., subjects with or without a diagnosis of disease or at different stages of disease or different subtypes of disease or different prognosis. Clinical trial subjects can be stratified into different groups to be treated the same or differently. Stratification can be based on any number of factors, including, stage of disease. Disease Staging is a classification system that uses diagnostic findings to produce clusters of patients based on such factors as etiology, pathophysiology and severity. It can serve as the basis for clustering clinically homogeneous patients to assess quality of care, analysis of clinical outcomes, utilization of resources, and the efficacy of alternative treatments.

In one method, potential clinical trial subjects are stratified at least in part on biomarker profiles of oligomeric and, optionally, monomeric forms of the protein biomarker (e.g., species of oligomeric and, optionally, monomeric alpha-synuclein; oligomeric and, optionally, monomeric amyloid beta, oligomeric and, optionally, monomeric tau; and oligomeric and, optionally, monomeric huntingtin). Thus, for example, subjects having different biomarker profiles (e.g., higher and lower relative amounts) can be assigned to different groups.

The population of subjects in a clinical trial should be sufficient to show whether the drug produces a statistically significant difference in outcome. Depending on this power level, the number of individuals in the trial can be at least 20, at least 100 or at least 500 subjects. Among these, there must be a significant number of individuals exhibiting a biomarker profile consistent with having the neurodegenerative condition (e.g., an increased level of the synuclein biomarker, i.e., relative level of oligomeric to monomeric alpha-synuclein). For example, at least 20%, at least 35%, at least 50%, or at least 66% of the subjects may initially have such a biomarker profile (comprising, e.g., various species of oligomeric and, optionally, monomeric alpha-synuclein; oligomeric and, optionally, monomeric amyloid beta, oligomeric and, optionally, monomeric tau; and oligomeric and, optionally, monomeric huntingtin). Also, a significant number of subjects are to be divided between class states. For example, at least 20%, at least 35%, at least 50%, at least 66% or 100% of the subjects may initially have a diagnosis of a neurodegenerative condition (e.g., synucleopathic condition (e.g., PD), amyloidopathic condition, tauopathic condition and Huntington's disease).

### B. Drug Development

Upon commencement of the clinical trial effectiveness of the therapeutic intervention on the different stratification groups can be rapidly determined as a function of the effect on the biomarker profile of the neurodegenerative protein (e.g., profiles of species of oligomeric and, optionally, monomeric alpha-synuclein; oligomeric and, optionally, monomeric amyloid beta, oligomeric and, optionally, monomeric tau; and oligomeric and, optionally, monomeric huntingtin). More specifically, a change in the biomarker profile of oligomeric and, optionally, monomeric forms of the protein predicts the clinical effectiveness of the therapeutic intervention. Methods generally involve first testing individuals to determine biomarker profile comprising oligomeric and, optionally, monomeric forms of the protein biomarker (e.g., species of oligomeric and, optionally, monomeric alpha-synuclein; oligomeric and, optionally, monomeric amyloid beta, oligomeric and, optionally, monomeric tau; and oligomeric and, optionally, monomeric huntingtin). After the measurements, the therapeutic intervention, e.g., an experimental drug, is administered to at least a subset of the subjects. Typically, at least a subset of the subjects is given a placebo or no treatment. In some cases, subject serve as their own controls, first receiving a placebo, and then, the experimental intervention, or the reverse, for comparison. In certain instances, this can be done in conjunction with administering already recognized forms of treatment. The population can be divided in terms of dosing, timing and rate of administration of the therapeutic intervention. Ethical considerations may require stopping a study when a statistically significant improvement is seen in test subjects. As used herein, "experimental drug" and "drug candidate" refer to an agent having or being tested for a therapeutic effect. A "putative neuroprotective agent" refers to an agent having or being tested to have neuroprotective action.

After administration of the therapeutic intervention, the biomarker profile is determined again.

The therapeutic intervention can be administration of a drug candidate. Using standard statistical methods, it can be determined whether the therapeutic intervention has had a meaningful impact on the biomarker profile comprising oligomeric and, optionally, monomeric forms of the protein biomarker (e.g., species of oligomeric and, optionally, monomeric alpha-synuclein; oligomeric and, optionally, monomeric amyloid beta, oligomeric and, optionally, monomeric tau; and oligomeric and, optionally, monomeric huntingtin). In general, a statistically significant change, especially a shift toward a normal profile, compared with the initial biomarker profile indicates that the therapeutic intervention is neuroprotective and thus will delay clinical onset, or slow or preferably reverse progression of the neurodegenerative condition (e.g., synucleopathic condition, amyloidopathic condition, tauopathic condition, Huntington's disease.

Accordingly, subjects for whom a biomarker profile comprising oligomeric to monomeric forms of the protein (e.g., species of oligomeric and, optionally, monomeric alpha-synuclein; oligomeric and, optionally, monomeric amyloid beta, oligomeric and, optionally, monomeric tau; and oligomeric and, optionally, monomeric huntingtin) (alternatively, oligomeric and total forms of the protein) can be measured include, for example: (1) Subjects who are asymptomatic for a neurodegenerative condition (e.g., synucleopathic condition, amyloidopathic condition, tauopathic condition, Huntington's disease); (2) subjects having minimal neurodegenerative disease symptoms and no signs suggestive of a neurodegenerative condition (e.g., who may be diagnosed with "suspected" or "preclinical" for a neurodegenerative condition, especially when certain genetic and/or environmental risk factors have been identified); (3) subjects having the diagnosis of "probable" neurodegenerative condition and subjects diagnosed ("definitive diagnosis") with a neurodegenerative condition. These include, for example, (1) subjects who are asymptomatic for a synucleopathic condition, (2) subjects having minimal parkinsonian symptoms and no signs suggestive of a synucleinopathic condition (e.g., who may be diagnosed with "suspected" or "preclinical" for PD or some related synucleinopathy, especially when certain genetic and/or environmental risk factors have been identified); (3) subjects having the diagnosis of "probable" synucleinopathy (e.g., PD) and subjects diagnosed ("definitive diagnosis") with a synucleinopathic condition.

Subjects are typically human but also include nonhuman animals, for example, those used as models for PD, such as, rodents (e.g., mice and rats), cats, dogs, other domesticated quadrupeds (such as horses, sheep and swine), and nonhuman primates (e.g., monkeys). Animal models include both genetic models and models based on the administration of neurotoxins. Neurotoxins used in such models include, for example, 6-hydroxydopamine (6-OHDA) and 1-methyl-1,2,3,6-tetrahydropyridine (MPTP) administration, and paraquat and rotenone. Genetic models include genetic mutations in SNCA (α-syn, PARK1, and 4), PRKN (parkin RBR E3 ubiquitin protein ligase, PARK2), PINK1 (PTEN-induced putative kinase 1, PARK6), DJ-1 (PARK7), and LRRK2 (leucine-rich repeat kinase 2, PARK8).

Clinical trials for neuroprotective therapies for neurodegenerative conditions, such as synucleinopathies require measures that promptly indicate the effectiveness of the potential therapy. Otherwise, the determination of drug efficacy based on clinical observations ordinarily takes many months. Biomarker profiles comprising neurodegenerative protein oligomers and, optionally monomers provide such measures, thus enabling the practical evaluation of disease modifying drug efficacy in subjects suffering from fatal brain disorders such as PD.

### VI. Methods of Treatment (not claimed)

Depending on the stage or class of neurodegenerative condition (e.g., synucleopathic condition, amyloidopathic condition, tauopathic condition, Huntington's disease) into which a subject is classified based on the biomarker profile as described herein, a subject may be in need of a therapeutic intervention. Provided herein are methods of treating a subject determined (not claimed), by the methods disclosed herein, to exhibit a neurodegenerative condition (e.g., a synucleopathic condition, and amyloidopathic condition, a tauopathic condition, Huntington's disease) with a therapeutic intervention effective to treat the condition. Therapeutic interventions that change and especially those that reduce the amount of oligomeric form of the protein biomarker to monomeric form of the protein biomarker (e.g., oligomeric and monomeric alpha-synuclein; oligomeric and monomeric amyloid beta, oligomeric and monomeric tau; and oligomeric and monomeric huntingtin), reflect an effective treatment, e.g., a therapeutic intervention developed by the methods herein, and clinically validated.

As used herein, the terms "therapeutic intervention", "therapy" and "treatment" refer to an intervention that produces a therapeutic effect, (e.g., is "therapeutically effective"). Therapeutically effective interventions prevent, slow the progression of, delay the onset of symptoms of, improve the condition of (e.g., causes remission of), improve symptoms of, or cure a disease, such as a synucleinopathic condition. A therapeutic intervention can include, for example, administration of a treatment, administration of a pharmaceutical, or a biologic or nutraceutical substance with therapeutic intent. The response to a therapeutic intervention can be complete or partial. In some aspects, the severity of disease is reduced by at least 10%, as compared, e.g., to the individual before administration or to a control individual not undergoing treatment. In some aspects the severity of disease is reduced by at least 25%, 50%, 75%, 80%, or 90%, or in some cases, no longer detectable using standard diagnostic techniques. Recognizing that certain sub-groups of subjects may not respond to a therapy, one measure of therapeutic effectiveness can be effectiveness for at least 90% of subjects undergoing the intervention over at least 100 subjects.

As used herein, the term "effective" as modifying a therapeutic intervention ("effective treatment" or "treatment effective to") or amount of a pharmaceutical drug ("effective amount"), refers to that treatment or amount to ameliorate a disorder, as described above. For example, for the given parameter, a therapeutically effective amount will show an increase or decrease in the parameter of at least 5%, 10%, 15%, 20%, 25%, 40%, 50%, 60%, 75%, 80%, 90%, or at least 100%. Therapeutic efficacy can also be expressed as "-fold" increase or decrease. For example, a therapeutically effective amount can have at least a 1.2-fold, 1.5-fold, 2-fold, 5-fold, or more effect over a control. Currently, clinically efficacy against the severity of motor symptoms in a parkinsonian subject can be measured using such standardized scales as the UPDRS and the Hoehn and Yahr scale; for metal and cognitive symptoms the ADAS-cog or the MMPI scales. (It is recognized that the utility of such scales does not necessarily depend on the type or nature of the underlying disease condition.)

Thus, according to some methods a subject is first tested for the biomarker profile comprising forms of oligomeric and/or monomeric forms of neurodegenerative proteins in a biological sample from the subject. A classification into an appropriate condition or class is determined based on the biomarker profile. Based on the classification a decision can be made regarding the type, amount, route and timing of administering an optimally effective therapeutic intervention to the subject.

### A. Synucleinopathic Condition

A symptom modifying therapeutic intervention for PD (i.e., a symptomatic or palliative treatment) may comprise administration of a drug selected from a dopamine agonist (e.g., pramipexole (e.g., Mirapex), ropinirole (e.g., Requip), rotigotine (e.g., Neupro), apomorphine (e.g., Apokyn)), levodopa, carbidopa-levodopa (e.g., Rytary, Sinemet), a MAO-B inhibitor (e.g., selegiline (e.g., Eldepryl, Zelapar) or rasagiline (e.g., Azilect)), a catechol-O-methyltransferase (COMT) inhibitor (e.g., entacapone (Comtan) or tolcapone (Tasmar)), an anticholinergic (e.g., benztropine (e.g., Cogentin) or trihexyphenidyl), amantadine or a cholinesterase inhibitor (e.g., rivastigmine (Exelon)) or some similar agent or group of agents (not claimed).

A neuroprotective or disease modifying therapeutic intervention for PD may comprise administration of a putatively disease modifying drug as described in any of the following provisional patent applications: Serial number 62/477187, filed March 27, 2017; Serial number 62/483,555, filed April 10, 2017; Serial number 62/485,082, filed April 13, 2017; Serial number 62/511,424, filed May 26, 2017; Serial number 62/528,228, filed July 3, 2017; Serial number 62/489,016, filed April 24, 2017; Serial number 62/527,215, filed June 30, 2017.

### B. Amyloidopathic Condition

A symptom modifying therapeutic intervention for an amyloidopathic condition (i.e., a symptomatic or palliative treatment) may comprise administration of a drug such as Razadyne^{®} (galantamine), Exelon^{®} (rivastigmine), and Aricept^{®} (donepezil) (not claimed).

### C. Tauopathic Condition

A symptom modifying therapeutic intervention for a tauopathic condition (i.e., a symptomatic or palliative treatment) may comprise administration of a drug such as Razadyne^{®} (galantamine), Exelon^{®} (rivastigmine), and Aricept^{®} (donepezil) or those cited herein used for the symptomatic treatment of PD (not claimed).

### D. Huntington's Disease

A symptom modifying therapeutic intervention for an Huntington's disease (i.e., a symptomatic or palliative treatment) may comprise administration of a drug such as tetrabenazine (Austedo^{®} (deutetrabenazine),IONIS-HTT_{Rx}, as well as various neuroleptics and benzodiazepines (not claimed).

### VII. Methods of Evaluating Responsiveness to Therapeutic Interventions

In a subject suffering from a neurodegenerative disorder (e.g., a synucleopathic condition, an amyloidopathic condition, a tauopathic condition, Huntington's disease) the effectiveness of a therapeutic intervention or the responsiveness of the subject to the therapeutic intervention can be determined by assessing the effect of the therapeutic intervention on the biomarker profile. This includes effectiveness in any neurodegenerative state, e.g., diagnosis, stage, progression, prognosis and risk. A change in the biomarker profile toward a more normal profile indicates effectiveness of the therapeutic intervention.

Use of biomarker profiles comprising oligomeric and, optionally, monomeric forms of a protein biomarker (e.g., species of oligomeric and, optionally, monomeric alpha-synuclein; oligomeric and, optionally, monomeric amyloid beta, oligomeric and, optionally, monomeric tau; and oligomeric and, optionally monomeric huntingtin), confers advantages over conventional means (e.g., changes in symptomatology, functional scales or radiologic scans) for judging treatment efficacy in such situations. Not only are such conventional means of judging efficacy insensitive, inexact and semi-quantitative, but typically require long periods (e.g., years) before becoming of sufficient magnitude to accurately measure. Accordingly, the number of potentially useful treatments tested is significantly reduced, and the expense of clinical trials and thus the eventual cost of useful medications is substantially increased

In certain embodiments, the biomarker profile of the protein biomarker species (e.g., alpha-synuclein, amyloid beta, tau or huntingtin) are measured a plurality of times, typically, before, during and after administration of the therapeutic intervention or at a plurality of time points after the therapeutic intervention.

### VIII. Kits (not claimed)

In another aspect, provided herein are kits for detecting oligomeric and monomeric protein biomarkers (e.g., species of oligomeric and monomeric alpha-synuclein; oligomeric and monomeric amyloid beta, oligomeric and monomeric tau; and oligomeric and monomeric huntingtin), and interpreting the results so obtained. The kits can comprise containers to hold reagents for isolating exosomes from a bodily fluid, reagents for preferentially isolated CNS-derived exosomes from all exosomes, first reagents sufficient to detect oligomeric forms of the protein biomarker (e.g., alpha-synuclein, amyloid beta, tau or huntingtin) and second reagents sufficient to detect a monomeric form of the protein biomarker (e.g., alpha-synuclein, amyloid beta, tau or huntingtin), or reagents to detect total protein biomarker species (e.g., alpha-synuclein, amyloid beta, tau or huntingtin).

For example, kits for use in detecting and staging a synucleinopathic disease state in a biological sample can comprise reagents, buffers, enzymes, antibodies and other compositions specific for this purpose. Kits can also typically include instructions for use as well as and software for data analysis and interpretation. The kit may further comprise samples that serve as normative standards. Each solution or composition may be contained in a vial or bottle and all vials held in close confinement in a box for commercial sale.

### EXAMPLES

The following examples are offered by way of illustration and not by way of limitation.

### Example 1: Alpha-synuclein oligomers are elevated compared with alpha-synuclein monomers in synucleopathic conditions

A cohort of individuals who have been diagnosed with a synucleopathic condition and are given an active therapeutic intervention and then one that is different, possibly known to be inactive, or the reverse, are the subject of study. Or a cohort comprising a plurality of subjects who are asymptomatic for a synucleopathic condition in a plurality of subjects who have been diagnosed with the synucleopathic condition are the subject of study. In either case, venous blood samples are is taken from each subject by venipuncture at various times, including under baseline or control (e.g., inactive intervention treatment) conditions and again during the administration of an potentially active (e.g., experimental intervention) treatment. CNS-derived exosomes are isolated from the blood using methods described herein. Amounts of monomeric alpha-synuclein and oligomeric alpha-synuclein or specific species thereof are measured that are contained within the isolated exosomes. A ratio of oligomeric alpha-synuclein species and to monomeric alpha-synuclein is determined. Results show that in the cohort of subjects diagnosed with the synucleopathic condition the ratio of oligomeric alpha-synuclein to monomeric alpha-synuclein is increased to a statistically significant degree. Those found to have a significant change in the results of this biomarker assay are later found to have a proportional change in clinical state.

### Example 2: Subject Stratification/Clinical Trial

Volunteer subjects without PD and with PD are tested to determine relative amounts of oligomeric and monomeric alpha-synuclein in CNS derived exosomes. Based on the relative amounts determined, and using cutoffs determined in the example above, the subjects are clustered into several test groups. Certain test groups are given a placebo. Other test groups are administered different amounts of a compound in a clinical trial. During and/or after administration, the tests are repeated. Collected measurements are analyzed. It is determined that the therapeutic intervention produces a statistically significant decrease in relative amounts of oligomeric alpha-synuclein to monomeric alpha-synuclein.

### Example 3: Clinical Trial for Drug Candidate That Is Neuroprotective for Synucleinopathies

The goal of a Phase II study is to evaluate the safety, tolerability and initial efficacy of pramipexole, given with Aprepitant and with or without and, optionally lovastatin or similarly effective drugs, in patients with PD and related disorders. A sequential treatment, rising-dose, cross-over, out-patient trial in up to 30 patients with PD (PD), Multiple system atrophy (MSA), Lewy body dementia (LBD), or related synucleopathic disorder is performed. None of the participants is allowed to have been treated with a dopamine agonist or other centrally active pharmaceutical during the 3 months prior to study entry, except for levodopa-carbidopa (Sinemet), which is maintained at a stable dose throughout the trial to a degree considered medically acceptable. Following baseline clinical and laboratory evaluations, including the United PD Rating Scale (UPDRS-Part III) and synuclein biomarker determinations, consenting individuals meeting accession criteria are switched from their pre-study PD treatment regimen to one that incudes pramipexole ER and Aprepitant. The pramipexole ER dose is titrated to that which is optimally tolerated (or a maximum of 9 mg/day) and then stably maintained for up to about 12 to16 weeks. Co-treatment with an additional drug (e.g., a statin) given at its maximum approved dose may then begin for an additional 3 months as deemed clinically appropriate, at which time all subjects are returned to their preadmission treatment regimen. During the trial, baseline efficacy and safety measures were repeated at regular intervals including determination of synuclein biomarker levels. Efficacy is determined as a function of statistically significant change toward normal of a biomarker profile comprising oligomeric alpha-synuclein and, optionally to monomeric alpha-synuclein species.

### Example 4: Diagnosis

A subject presents having certain symptoms consistent with PD but, at a preclinical level when still lacking many of the distinguishing clinical features of this illness. Blood is taken from the subject through venipuncture. Amounts of oligomeric and monomeric alpha-synuclein are measured from CNS-derived exosomes in the blood. A biomarker profile is determined. A diagnostic algorithm classifies the profile to be consistent with a diagnosis of PD. The subject is diagnosed with PD, and is placed on a therapeutic regimen, either a palliative to mitigate symptoms, or treatment directed to the etiology of the disease for purposes of neuroprotection.

### Example 5: Staging

A subject presents with a diagnosis of PD. The doctor orders a blood test on the subject to determine a biomarker profile comprising oligomeric and, optionally, monomeric alpha-synuclein. Based on the biomarker profile comprising oligomeric alpha-synuclein and, optionally monomers, the doctor determines that the subject is at an early stage of PD and thus more responsive to a particular therapeutic intervention.

### Example 6: Prognosis/Progression

A subject presents with a diagnosis of PD. The doctor orders first and second blood tests on the subject several months apart to determine a biomarker profile comprising oligomeric and, optionally, monomeric alpha-synuclein. Based on the biomarker profile oligomeric alpha-synuclein to monomeric, the doctor determines that the subject's disease is progressing slowly and that the subject is expected to have many years of useful life, even without a risky therapeutic intervention.

### Example 7: Risk Assessment

A subject presents for a physical exam having no symptoms of a synucleinopathic disease. In this case, this individual is aware of a genetic or environmental risk factor. The doctor orders a blood test on the subject to determine a biomarker profile comprising oligomeric and, optionally, monomeric alpha-synuclein. Based on the relatively abnormal biomarker profile of some or all measurable species of oligomeric alpha-synuclein, compared to healthy control individuals, the doctor determines that the subject has a low probability of developing PD.

### Example 8: Response to Therapy

A subject presents with a diagnosis of PD. The doctor orders initial blood tests on the subject to determine a biomarker profile comprising oligomeric and, optionally, monomeric alpha-synuclein before treatment commences. After a round of treatment, but before clinical symptoms have changed, the doctor orders a second blood test. Based on a change towards normal in the a, the doctor determines that the treatment is effective or whether the dose needs to be changed or repeated.

### Example 9: Development of Diagnostic

Volunteer subjects without PD and with PD at different diagnosed stages are tested to determine a biomarker profile comprising a plurality of oligomeric alpha-synuclein and monomeric alpha-synuclein. Based on the biomarker profile determined, subjects are classified as showing presence or absence of disease and, optionally stage of disease. Profiles are determined using a computerized learning algorithm that, after data analysis, generates a classification algorithm that infers a diagnosis. The inference model is selected to produce a test with a desired sensitivity and specificity.

### Example 10: Alpha-synuclein oligomer profiles are changed in synucleopathic conditions

A cohort of individuals who are the subject of study have been diagnosed with a synucleopathic condition. The subjects are given an active therapeutic intervention and then one that is different, possibly known to be inactive. Alternatively, the interventions can be given in the reverse order. Or a cohort comprising a plurality of subjects who are asymptomatic for a synucleopathic condition in a plurality of subjects who have been diagnosed with the synucleopathic condition are the subject of study. In either case, venous blood samples are is taken from each subject by venipuncture at various times, including under baseline or control (e.g., inactive intervention treatment) conditions and again during the administration of a potentially active (e.g., experimental intervention) treatment. CNS-derived exosomes are isolated from the blood using methods described herein. Amounts of a plurality of alpha-synuclein forms, including monomeric alpha-synuclein and oligomeric alpha-synuclein that are contained within the isolated exosomes are measured. These data are combined into a dataset. The dataset is analyzed using statistical methods, in this case, used to train a learning algorithm, e.g., a support vector machine, to develop a model that infers whether a subject should be classified as having or not having the synucleopathic condition. Results show that in the cohort of subjects diagnosed with the synucleopathic condition certain species of oligomeric alpha-synuclein are increased to a statistically significant degree relative to other oligomeric species and, optionally, monomeric species. Those found to have a significant change in the results of this biomarker assay are later found to have a proportional change in clinical state.

### Example 11: Subject Stratification/Clinical Trial

Volunteer subjects without PD and with PD are tested to determine a biomarker profile of oligomeric and, optionally, monomeric alpha-synuclein in CNS derived exosomes. Based on the biomarker profile determined, and using a classifier determined in the example above, the subjects are clustered into several test groups. Certain test groups are given a placebo. Other test groups are administered different amounts of a compound in a clinical trial. During and, optionally after administration, the tests are repeated. Collected measurements are analyzed. It is determined that the therapeutic intervention produces a statistically significant change toward normal of biomarker profiles comprising oligomeric alpha-synuclein and, optionally, monomeric alpha-synuclein.

### Example 12: Clinical Trial for Drug Candidate That Is Neuroprotective for Synucleinopathies

The goal of a Phase II study is to evaluate the safety, tolerability and initial efficacy of pramipexole, given with Aprepitant and with or without and, optionally lovastatin or similarly effective drugs, in patients with PD and related disorders. A sequential treatment, rising-dose, cross-over, out-patient trial in up to 30 patients with PD (PD), Multiple system atrophy (MSA), Lewy body dementia (LBD), or related synucleopathic disorder is performed. None of the participants is allowed to have been treated with a dopamine agonist or other centrally active pharmaceutical during the 3 months prior to study entry, except for levodopa-carbidopa (Sinemet), which is maintained at a stable dose throughout the trial to a degree considered medically acceptable. Following baseline clinical and laboratory evaluations, including the United PD Rating Scale (UPDRS-Part III) and synuclein biomarker determinations, consenting individuals meeting accession criteria are switched from their pre-study PD treatment regimen to one that incudes pramipexole ER and Aprepitant. The pramipexole ER dose is titrated to that which is optimally tolerated (or a maximum of 9 mg/day) and then stably maintained for up to about 12 to16 weeks. Co-treatment with an additional drug (e.g., a statin) given at its maximum approved dose may then begin for an additional 3 months as deemed clinically appropriate, at which time all subjects are returned to their preadmission treatment regimen. During the trial, baseline efficacy and safety measures were repeated at regular intervals including determination of synuclein biomarker levels. Efficacy is determined as a function of statistically significant change toward normal of a biomarker profile comprising oligomeric alpha-synuclein and, optionally to monomeric alpha-synuclein species.

### Example 13: Diagnosis

A subject presents having certain symptoms consistent with PD but, at a preclinical level when still lacking many of the distinguishing clinical features of this illness. Blood is taken from the subject through venipuncture. Amounts of oligomeric and monomeric alpha-synuclein are measured from CNS-derived exosomes in the blood. A biomarker profile is determined. A diagnostic algorithm classifies the profile to be consistent with a diagnosis of PD. The subject is diagnosed with PD, and is placed on a therapeutic regimen, either a palliative to mitigate symptoms, or treatment directed to the etiology of the disease for purposes of neuroprotection.

### Example 14: Staging

A subject presents with a diagnosis of PD. The doctor orders a blood test on the subject to determine a biomarker profile comprising oligomeric and, optionally, monomeric alpha-synuclein. Based on the biomarker profile comprising oligomeric alpha-synuclein and, optionally monomers, the doctor determines that the subject is at an early stage of PD and thus more responsive to a particular therapeutic intervention.

### Example 15: Prognosis/Progression

A subject presents with a diagnosis of PD. The doctor orders first and second blood tests on the subject several months apart to determine a biomarker profile comprising oligomeric and, optionally, monomeric alpha-synuclein. Based on the biomarker profile oligomeric alpha-synuclein to monomeric, the doctor determines that the subject's disease is progressing slowly and that the subject is expected to have many years of useful life, even without a risky therapeutic intervention.

### Example 16: Risk Assessment

A subject presents for a physical exam having no symptoms of a synucleinopathic disease. In this case, this individual is aware of a genetic or environmental risk factor. The doctor orders a blood test on the subject to determine a biomarker profile comprising oligomeric and, optionally, monomeric alpha-synuclein. Based on the relatively abnormal biomarker profile of some or all measurable species of oligomeric alpha-synuclein, compared to healthy control individuals, the doctor determines that the subject has a low probability of developing PD.

### Example 17: Response to Therapy

A subject presents with a diagnosis of PD. The doctor orders initial blood tests on the subject to determine a biomarker profile comprising oligomeric and, optionally, monomeric alpha-synuclein before treatment commences. After a round of treatment, but before clinical symptoms have changed, the doctor orders a second blood test. Based on a change towards normal in the a, the doctor determines that the treatment is effective or whether the dose needs to be changed or repeated.

### Example 18: Exemplary Biomarker Profiles

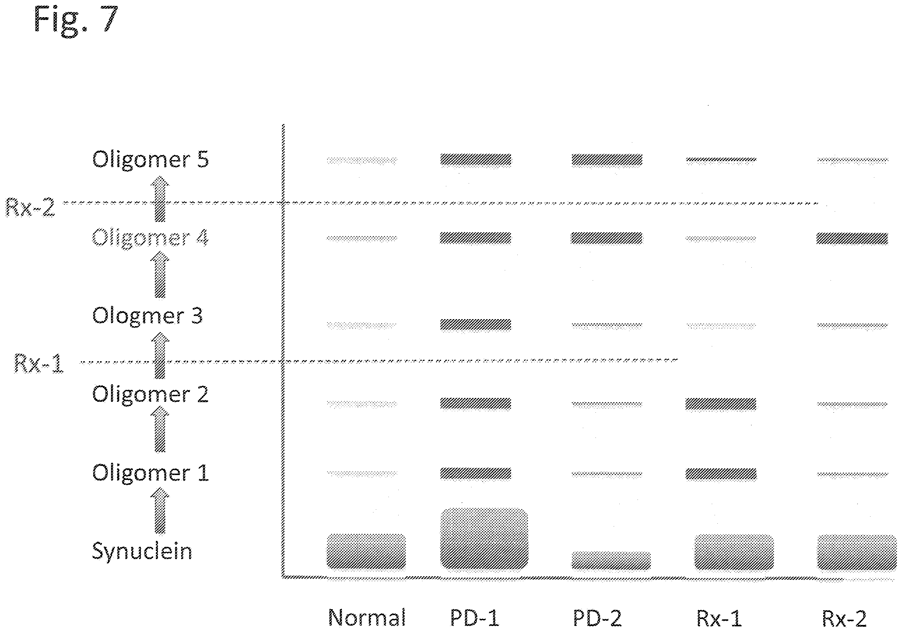

**FIG. 7** shows exemplary biomarker profiles including monomeric and five oligomeric species of alpha synuclein in five different states. The states include normal, Parkinson's disease stage 1 (PD-1), Parkinson's disease stage 2 (PD-2), treatment with therapeutic agent 1 (Rx-1) and treatment with therapeutic agent 2 (Rx-2). Relative amounts of each of oligomeric species are indicated by darkness of the line. As can be seen, oligomer 4 is elevated in both Stage 1 and Stage 2 Parkinson's disease. In contrast, oligomers 1, 2 and 3 are elevated in Stage 1 but not Stage 2. Therapeutic agent 1 reduces relative amounts of oligomer 4 and is considered to have neuroprotective activity. By comparison, therapeutic agent 2 does not reduce oligomer 4 and, in this example, is considered not to be neuroprotective.

### Example 19: Development of Diagnostic for Alzheimer's Disease

Volunteer subjects diagnosed by a medical professional to have Alzheimer's disease or not to have Alzheimer's disease provide blood samples for testing. The internal contents of brain-derived exosomes are isolated. Amounts of monomeric α-beta and each of a plurality of species of oligomeric α-beta are determined. Comparison of the results shows that in subjects diagnosed with Alzheimer's disease, one oligomeric form is consistently increased as compared with monomeric α-beta. It is further determined that an amount of this form above a determined threshold level provides a diagnosis of Alzheimer's disease with 85% sensitivity and 98% specificity. This threshold level is used to diagnose other subjects with Alzheimer's disease.

### Example 19: Development of Diagnostic for Huntington's Disease

Volunteer subjects diagnosed by a medical professional to have Huntington's disease or not to have Huntington's disease provide blood samples for testing. The internal contents of brain-derived exosomes are isolated. Amounts of each of a plurality of species of oligomeric huntingtin protein are determined. Using a linear regression analysis, it is found that amounts of three separate oligomeric forms in combination, can diagnose Huntington's disease in the form of a linear mathematical model.

As used herein, the following meanings apply unless otherwise specified. The word "may" is used in a permissive sense (i.e., meaning having the potential to), rather than the mandatory sense (i.e., meaning must). The words "include", "including", and "includes" and the like mean including, but not limited to. The singular forms "a," "an," and "the" include plural referents. Thus, for example, reference to "an element" includes a combination of two or more elements, notwithstanding use of other terms and phrases for one or more elements, such as "one or more." The term "or" is, unless indicated otherwise, non-exclusive, i.e., encompassing both "and" and "or." The term "any of" between a modifier and a sequence means that the modifier modifies each member of the sequence. So, for example, the phrase "at least any of 1, 2 or 3" means "at least 1, at least 2 or at least 3".The phrase "at least one" includes "a plurality".

While certain embodiments of the present invention have been shown and described herein, it will be obvious to those skilled in the art that such embodiments are provided by way of example only.

## Claims

1. A method of inferring a risk of developing, a diagnosis of, a stage of, a prognosis of, a progression of, or a degree of response to a putative neuroprotective agent for a synucleinopathy, wherein the method comprises:
a) determining, from a blood sample from a subject that is enriched for CNS-derived microsomal particles, an alpha-synuclein profile comprising quantitative measures of one or more oligomeric forms of alpha-synuclein and, optionally, a monomeric form of alpha-synuclein to create a test dataset; and
b) executing a model on the test dataset to infer a risk of developing, a diagnosis of, a stage of, a prognosis of, a progression of, or a degree of response to a putative neuroprotective agent for the synucleinopathy, wherein the model is obtained by:
(I) providing a training dataset comprising, for each of a plurality of subjects, values indicating (1) state of a synucleinopathy or response to putative neuroprotective agent for a synucleinopathy , and (2) quantitative measures of amounts of each of one or more oligomeric forms of alpha-synuclein and, optionally, a monomeric form of alpha-synuclein in a blood sample enriched for CNS-derived microsomal particles; and
(II) performing a statistical analysis on the training dataset to develop a model that infers the state of the synucleinopathy in an individual.

2. The method of claim 1, wherein the alpha-synuclein forms for which the quantitative measures are determined are selected from:
(I) at least one oligomeric form;
(II) a plurality of oligomeric forms;
(III) at least one oligomeric form and at least one monomeric form;
(IV) a plurality of oligomeric forms and at least one monomeric form;
(V) at least one oligomeric form and a plurality of monomeric forms;
(VI) a plurality of oligomeric forms and a plurality of monomeric forms; and
(VII) at least one range of sizes of oligomeric forms.

3. The method of any one of the preceding claims, wherein at least one of the oligomeric forms comprises a collection of species of the alpha-synuclein.

4. The method of any one of the preceding claims, wherein the model comprises: correlational, Pearson correlation, Spearman correlation, chisquare, comparison of means (e.g., paired T-test, independent T-test, ANOVA regression analysis (e.g., simple regression, multiple regression, linear regression, non-linear regression, logistic regression, polynomial regression, stepwise regression, ridge regression, lasso regression, elasticnet regression) or non-parametric analysis (e.g., Wilcoxon rank-sum test, Wilcoxon sign-rank test, sign test).

5. The method of any one of the preceding claims, wherein the model is executed by computer.

6. The method of any one of the preceding claims, wherein the synucleinopathy is selected from Parkinson's disease, Lewy body dementia, multiple system atrophy or a related disorder.

7. The method of any one of the preceding claims, wherein the synucleinopathy is Parkinson's disease.

8. The method of any one of the preceding claims, wherein the oligomeric forms include one or more relatively low molecular weight synuclein oligomers.

9. The method of any one of the preceding claims, wherein the oligomeric synuclein forms include oligomeric forms in a size range of about 6-mers to 18-mers.

10. The method of any one of the preceding claims, wherein the blood sample comprises a venous blood sample.

11. The method of any one of the preceding claims, wherein the CNS-derived microsomal particles are washed to remove surface membrane-bound proteins.

12. The method of any one of the preceding claims, wherein the model uses a pattern of relative amounts of a plurality of the oligomeric forms of alpha-synuclein or a ratio of oligomeric alpha-synuclein forms to monomeric alpha-synuclein.

13. The method of any one of the preceding claims, wherein one or more oligomeric forms of alpha-synuclein and, optionally, a monomeric form of alpha-synuclein are isolated from an internal compartment of the microsomal particles.

## Patentansprüche

1. Verfahren zum Ableiten eines Entwicklungsrisikos, einer Diagnose, eines Stadiums, einer Prognose, eines Fortschreitens einer Synucleinopathie oder eines Grads des Ansprechens auf ein mutmaßliches neuroprotektives Mittel für diese, wobei das Verfahren Folgendes umfasst:
a) Bestimmen eines alpha-Synuclein-Profils, das quantitative Messungen einer oder mehrerer oligomerer Formen von alpha-Synuclein und optional einer monomeren Form von alpha-Synuclein umfasst, aus einer Blutprobe von einem Subjekt, die mit aus dem ZNS stammenden mikrosomalen Partikeln angereichert ist, um einen Testdatensatz zu erzeugen; und
b) Ausführen eines Modells an dem Testdatensatz, um ein Entwicklungsrisiko, eine Diagnose, ein Stadium, eine Prognose, ein Fortschreiten der Synucleinopathie oder einen Grad des Ansprechens auf ein mutmaßliches neuroprotektives Mittel für diese abzuleiten, wobei das Modell durch Folgendes erlangt wird:
(I) Bereitstellen eines Trainingsdatensatzes, der für jedes von einer Vielzahl von Subjekten Werte umfasst, die (1) einen Zustand einer Synucleinopathie oder ein Ansprechen auf ein mutmaßliches neuroprotektives Mittel für eine Synucleinopathie und (2) quantitative Maße von Mengen von jeder von einer oder mehreren oligomeren Formen von alpha-Synuclein und optional einer monomere Form von alpha-Synuclein in einer Blutprobe, die mit aus dem ZNS stammenden mikrosomalen Partikeln angereichert ist, angeben; und
(II) Durchführen einer statistischen Analyse an dem Trainingsdatensatz, um ein
Modell zu entwickeln, aus dem der Zustand der Synucleinopathie bei einem Individuum abzuleiten ist.

2. Verfahren nach Anspruch 1, wobei die Alpha-Synuclein-Formen, für die die quantitativen Maße bestimmt werden, ausgewählt sind aus:
(I) mindestens einer oligomeren Form;
(II) einer Vielzahl von oligomeren Formen;
(III) mindestens einer oligomeren Form und mindestens einer monomeren Form;
(IV) einer Vielzahl von oligomeren Formen und mindestens einer monomeren Form;
(V) mindestens einer oligomeren Form und einer Vielzahl von monomeren Formen;
(VI) einer Vielzahl von oligomeren Formen und einer Vielzahl von monomeren Formen; und
(VII) mindestens einen Größenbereich von oligomeren Formen.

3. Verfahren nach einem der vorhergehenden Ansprüche, wobei mindestens eine der oligomeren Formen eine Sammlung von Spezies des alpha-Synucleins umfasst.

4. Verfahren nach einem der vorhergehenden Ansprüche, wobei das Modell umfasst: Korrelation, Pearson-Korrelation, Spearman-Korrelation, Chi-Quadrat, Mittelwertvergleich (z. B. gepaarter T-Test, unabhängiger T-Test, ANOVA-Regressionsanalyse (z. B. einfache Regression, multiple Regression, lineare Regression, nichtlineare Regression, logistische Regression, polynomiale Regression, schrittweise Regression, Ridge-Regression, Lasso-Regression, Elasticnet-Regression) oder nichtparametrische Analyse (z. B. Wilcoxon-Rangsummentest, Wilcoxon-Vorzeichen-Rangtest, Vorzeichentest).

5. Verfahren nach einem der vorhergehenden Ansprüche, wobei das Modell durch einen Computer ausgeführt wird.

6. Verfahren nach einem der vorhergehenden Ansprüche, wobei die Synucleinopathie ausgewählt ist aus Parkinson-Krankheit, Lewy-Körper-Demenz, Multisystematrophie oder einer verwandten Störung.

7. Verfahren nach einem der vorhergehenden Ansprüche, wobei die Synucleinopathie die Parkinson-Krankheit ist.

8. Verfahren nach einem der vorhergehenden Ansprüche, wobei die oligomeren Formen ein oder mehrere Synuclein-Oligomere mit relativ niedrigem Molekulargewicht enthalten.

9. Verfahren nach einem der vorhergehenden Ansprüche, wobei die oligomeren Synuclein-Formen oligomere Formen in einem Größenbereich von etwa 6-meren bis 18-meren enthalten.

10. Verfahren nach einem der vorhergehenden Ansprüche, wobei die Blutprobe eine venöse Blutprobe umfasst.

11. Verfahren nach einem der vorhergehenden Ansprüche, wobei die aus dem ZNS stammenden mikrosomalen Partikel gewaschen werden, um oberflächenmembrangebundene Proteine zu entfernen.

12. Verfahren nach einem der vorhergehenden Ansprüche, wobei das Modell ein Muster relativer Mengen einer Vielzahl der oligomeren Formen von alpha-Synuclein oder ein Verhältnis von oligomeren alpha-Synuclein-Formen zu monomerem alpha-Synuclein verwendet.

13. Verfahren nach einem der vorhergehenden Ansprüche, wobei eine oder mehrere oligomere Formen von alpha-Synuclein und optional eine monomere Form von alpha-Synuclein aus einem inneren Kompartiment der mikrosomalen Partikel isoliert werden.

## Revendications

1. Procédé permettant de déduire un risque de développer, un diagnostic, un stade, un pronostic, une progression ou un degré de réponse à un agent neuroprotecteur putatif pour une synucléinopathie, ledit procédé comprenant :
a) la détermination, à partir d'un échantillon de sang d'un sujet qui est enrichi en particules microsomales dérivées du SNC, d'un profil d'alpha-synucléine comprenant des mesures quantitatives d'une ou plusieurs formes oligomères d'alpha-synucléine et, éventuellement, d'une forme monomère d'alpha-synucléine pour créer un ensemble de données de test ; et
b) l'exécution d'un modèle sur l'ensemble de données de test pour déduire un risque de développement, un diagnostic, un stade, un pronostic, une progression ou un degré de réponse à un agent neuroprotecteur putatif pour la synucléinopathie, ledit modèle étant obtenu par :
(I) la fourniture d'un ensemble de données d'apprentissage comprenant, pour chacun d'une pluralité de sujets, des valeurs indiquant (1) l'état d'une synucléinopathie ou la réponse à un agent neuroprotecteur putatif pour une synucléinopathie, et (2) les mesures quantitatives de quantités de chacune d'une ou plusieurs formes oligomères de l'alpha-synucléine et, éventuellement, d'une forme monomère d'alpha-synucléine dans un échantillon de sang enrichi en particules microsomales dérivées du SNC ; et
(II) la réalisation d'une analyse statistique sur l'ensemble de données d'apprentissage pour développer un modèle qui déduit l'état de la synucléinopathie chez un individu.

2. Procédé selon la revendication 1, lesdites formes d'alpha-synucléine pour lesquelles les mesures quantitatives sont déterminées étant choisies parmi :
(I) au moins une forme oligomère ;
(II) une pluralité de formes oligomères ;
(III) au moins une forme oligomère et au moins une forme monomère ;
(IV) une pluralité de formes oligomères et au moins une forme monomère ;
(V) au moins une forme oligomère et une pluralité de formes monomères ;
(VI) une pluralité de formes oligomères et une pluralité de formes monomères ; et
(VII) au moins une plage de tailles de formes oligomères.

3. Procédé selon l'une quelconque des revendications précédentes, au moins l'une desdites formes oligomères comprenant une collection d'espèces de l'alpha-synucléine.

4. Procédé selon l'une quelconque des revendications précédentes, ledit modèle comprenant : une corrélation, la corrélation de Pearson, la corrélation de Spearman, le chi carré, la comparaison des moyennes (par ex., test T pour échantillons appariés, test T pour échantillons indépendants, l'analyse de régression ANOVA (par ex., simple régression, régression multiple, régression linéaire, régression non linéaire, régression logistique, régression polynomiale, régression pas à pas, régression ridge, régression lasso, régression elasticnet) ou une analyse non paramétrique (par ex., test de la somme des rangs de Wilcoxon, test des rangs signés de Wilcoxon, test des signes).

5. Procédé selon l'une quelconque des revendications précédentes, ledit modèle étant exécuté par ordinateur.

6. Procédé selon l'une quelconque des revendications précédentes, ladite synucléinopathie étant choisie parmi la maladie de Parkinson, la démence à corps de Lewy, l'atrophie multisystématisée ou un trouble apparenté.

7. Procédé selon l'une quelconque des revendications précédentes, ladite synucléinopathie étant la maladie de Parkinson.

8. Procédé selon l'une quelconque des revendications précédentes, lesdites formes oligomères comprenant un ou plusieurs oligomères de synucléine de masse moléculaire relativement faible.

9. Procédé selon l'une quelconque des revendications précédentes, lesdites formes oligomères de synucléine comprenant les formes oligomères dans une plage de taille d'environ 6-mères à 18-mères.

10. Procédé selon l'une quelconque des revendications précédentes, ledit échantillon de sang comprenant un échantillon de sang veineux.

11. Procédé selon l'une quelconque des revendications précédentes, lesdites particules microsomales dérivées du SNC étant lavées pour éliminer les protéines de surface liées à la membrane.

12. Procédé selon l'une quelconque des revendications précédentes, ledit modèle utilisant un motif de quantités relatives d'une pluralité de formes oligomères d'alpha-synucléine ou le rapport des formes oligomères d'alpha-synucléine à l'alpha-synucléine monomère.

13. Procédé selon l'une quelconque des revendications précédentes, une ou plusieurs formes oligomères d'alpha-synucléine et, éventuellement, une forme monomère d'alpha-synucléine étant isolées d'un compartiment interne des particules microsomales.
